# EUROPEAN PATENT SPECIFICATION

(11) **EP 2 420 830 B1**
(45) Date of publication and mention of the grant of the patent: **16.11.2016**
(21) Application number: 11177708.2
(22) Date of filing: 16.08.2011
(51) Int. Cl.: G01N 33/50, C07K 14/705

(54) **Chloride transporter ClC-7 and cell-based screening method**
Chloridtransporter ClC-7 und zellbasiertes Screening-Verfahren
Transporteur CIC-7 de chlorure et procédé de criblage à base cellulaire

(30) Priority: 16.08.2010 EP 10075351
(43) Date of publication of application: 22.02.2012
(73) Proprietor: Forschungsverbund Berlin e.V., 12489 Berlin (DE); Max-Delbrück-Centrum für Molekulare Medizin in der Helmholtz-Gemeinschaft, 13125 Berlin (DE)
(72) Inventor: Stauber, Tobias, 10439 Berlin (DE); Jentsch, Thomas, 10405 Berlin (DE)
(74) Representative: Hertin und Partner Rechts- und Patentanwälte PartG mbB

(56) References cited:
- WO-A1-03/062821
- DE-A1- 10 102 977
- Z. ZHAO ET AL: "The ClC-3 Chloride Transport Protein Traffics through the Plasma Membrane via Interaction of an N-terminal Dileucine Cluster with Clathrin", JOURNAL OF BIOLOGICAL CHEMISTRY, vol. 282, no. 39, 1 September 2007 (2007-09-01), pages 29022-29031, XP55014700, ISSN: 0021-9258, DOI: 10.1074/jbc.M703506200
- T. STAUBER ET AL: "Sorting Motifs of the Endosomal/Lysosomal CLC Chloride Transporters", JOURNAL OF BIOLOGICAL CHEMISTRY, vol. 285, no. 45, 5 November 2010 (2010-11-05), pages 34537-34548, XP55014516, ISSN: 0021-9258, DOI: 10.1074/jbc.M110.162545
- LILIA LEISLE ET AL: "ClC-7 is a slowly voltage-gated 2Cl-/1H+-exchanger and requires Ostm1 for transport activity", THE EMBO JOURNAL, vol. 30, no. 11, 1 June 2011 (2011-06-01), pages 2140-2152, XP55014511, ISSN: 0261-4189, DOI: 10.1038/emboj.2011.137
- DATABASE UNIPROT [Online] 05 July 2005 'SubName: Full=Chloride channel 7; Flags: Fragment;' Retrieved from EBI, accession no. UNIPROT:Q4VXY0 Database accession no. Q4VXY0

## Description

The invention relates to a CIC-7 protein that is presented on the cell surface and/or plasma membrane, characterised in that the interaction between CIC-7 and the endosomal and/or lysosomal sorting machinery, such as one or more adaptor proteins (AP) and/or Golgi-localized, γ-ear containing, Arf-binding adaptor proteins (GGA), is inhibited and/or disrupted, preferably by mutation or deletion of the amino acid sequences of one or more AP and/or GGA recognition motifs. The invention further relates to a cell-based assay system in which a CIC-7 protein is presented on the cell surface and/or plasma membrane of a cell, with which test compounds are screened for activity in modulating proton and/or chloride transport via CIC-7. Such compounds are intended for application in the treatment of osteoporosis.

### BACKGROUND OF THE INVENTION

The CLC protein family comprises of plasma membrane chloride channels and the intracellular chloride-proton exchangers CIC-3 through -7. CIC-7 does not provide pure chloride conductance, but its chloride transport is tightly coupled to a transport of protons in the opposite direction (antiport) (7, 54). These intracellular CLC proteins mainly reside on the various compartments of the endosomal/lysosomal system where they are involved in the luminal acidification or chloride accumulation. Additionally, CIC-7 also localises to the ruffled border of osteoclasts, which are the bone cells responsible for bone resorption. The ruffled border is a specialised membrane region of osteoclasts, formed mainly through lysosomal exocytosis, and which acidifies the resorption lacunae by secreting protons. The resorption lacunae represent a limited space between the osteoclast and bone in which bone material is dismantled and reabsorbed.

It is known that mutations in CIC-7, which lead to loss of protein, changes in ion transport activity or other defects, result in an increased mineralisation of the bone material, a process named osteopetrosis. There have been approximately 50 mutations described in *CLCN7,* the human gene encoding CIC-7, which can lead to osteopetrosis or related conditions of unwanted bone mineralisation.

The inhibition of CIC-7 therefore represents a promising target for the treatment of osteoporosis, a condition related to insufficient bone strength that occurs frequently in patients of older age. Bone formation and resorption are in constant flux and balance, mediated via the actions of osteoblasts and osteoclasts. Osteopetrosis and osteoporosis are caused by an imbalance of biologically controlled processes that maintain appropriate bone mineralisation and density.

EP 1 360 504 discloses a method for screening chemical compounds for potential activity in the treatment, prevention, or alleviation of an osteoclasts related bone disease in a subject, which method comprises screening compounds in a test cell so as to select compounds having the ability to block one or more channels or transporters of the CIC family. Substances identified by such a screen, which inhibit chloride transporters of the CIC family, could be applied as treatments for osteoporosis, thereby blocking CIC proteins and increasing bone strength.

A similar effect to CIC-7 inhibition can also be achieved via inhibition of the physical interaction between CIC-7 and its interaction partner Ostm1. It has been shown that Ostm1 knock-out mice also lack CIC-7, whereby Ostm1 and CIC-7 knock-out mice exhibit the same strong osteopetrosis phenotype, as the two proteins stabilise one another (38).

EP 1 963 864 discloses a method for screening test compounds for activity in modulating chloride transport via CIC-7 or for modulating subcellular localisation of Ostm 1, whereby the screening assay is able to measure whether or to what extent the CIC-7-Ostm1 interaction is disrupted. The disruption of the CIC-7-Ostm1 interaction is functionally related to the inhibition of CIC-7, therefore such compounds could also be applied as treatments for osteoporosis.

However, a major disadvantage of the two methods already proposed for using CIC-7 and/or Ostm1 to identify potential compounds for the treatment, prevention, or alleviation of an osteoclast-related bone disease is the sub-cellular localisation of CIC-7. Despite the known role of CIC-7 and Ostm1 in regulating bone formation and resorption, the screening of compounds that potentially inhibit CIC-7 or its interaction with Ostm1 is significantly impeded by the sub-cellular localisation of CIC-7. Because CIC-7 mainly resides in the various compartments of the endosomal/lysosomal system, methods for testing or identifying CIC-7-inhibiting compounds has been difficult. Firstly, the CIC-7 protein is not sufficiently accessible to test compounds, so potential inhibitors of CIC-function must not only inhibit the chloride transporter but additionally pass through the plasma membrane and interact with the chloride transporter in a very limited and potentially difficult to access sub-cellular space. Secondly, due to the restriction of the CIC-7 transporter to lysosomal or endosomal membranes, a very limited number of assay methods are available for detecting changes in ion transport due to inhibited CIC-7 action. Measurement of changes in lysosomal or endosomal pH represents a difficult protocol poorly suited for a high-throughput screening methods. Moreover, lack of CIC-7 does not change lysosomal steady-state pH (7, 38, 39).

These factors represent an enormous barrier to developing an effective test system for screening compounds for CIC-7-inhibitory action.

Until now the expression of CIC-7 on the cell surface has not been possible. Overexpression studies also demonstrate that even when overexpressed CIC-7 does not localise to the cell surface and/or cell membrane.

Although the partially overlapping subcellular distribution of the CIC-3 through -7 proteins has been studied extensively, little is known about their targeting mechanism. Using a comprehensive approach we performed pull-down experiments to systematically map the differential binding of adaptor proteins of the endosomal sorting machinery (APs and GGAs), as well as clathrin, to the cytosolic regions of the intracellular CLCs. The resulting interaction pattern fitted well to the known subcellular localizations of the CLCs. By mutating potential sorting motifs, we could locate almost all binding sites, including one already known for CIC-3 and several new motifs for CIC-5, -6 and -7. The impact of the identified binding sites on the subcellular localization of CLC transporters was determined by heterologous expression of mutants. Surprisingly, some vesicular CLCs retained their localization after disruption of interaction sites. However, CIC-7 could be partially shifted from lysosomes to the plasma membrane by combined mutation of N-terminal sorting motifs. The localization of its β-subunit, Ostm1, was determined by that of CIC-7. Ostm1 was not capable of redirecting CIC-7 to lysosomes.

The CLC family of chloride transport proteins comprises nine members in mammals (1). While four of these are plasma membrane-residing chloride channels, the other five, CIC-3 through - 7, localize to distinct, yet partially overlapping compartments of the endo-lysosomal pathway and to other specialized vesicles of the late biosynthetic pathway like synaptic vesicles (1-5). On these organelles, they are involved in enabling luminal acidification and/or chloride accumulation (6-10). Despite the pivotal role of CLC proteins in endo-lysosomal function and their involvement in diverse pathologies in mouse models and human genetic disease (1), little is known about the sorting steps by which they reach their subcellular destinations.

Sorting of endo-lysosomal transmembrane proteins is usually mediated by cytosolic motifs that are recognized by adaptor proteins. These recruit further components of the protein transport machinery, such as clathrin (11,12). The dileucine motif [DE]XXXL[LI] and the tyrosine-based motif YXXΦ (with Φ being a bulky, hydrophobic amino acid) are recognized by the adaptor protein (AP) complexes AP-1 through -4. The cell uses different APs to recruit cargo proteins for specific transport routes, such as AP-2 for endocytosis from the plasma membrane, AP-1 for transport between the *trans*-Golgi network (TGN) and early endosomes, and AP-3 for sorting from early endosomes and the TGN to late endosomes (13). The role of AP-4 is less understood. Another sorting signal, which is recognized by adaptors of the Golgi-localized, γ-ear containing, Arf-binding (GGA) family, is the dileucine motif DXXLL (14). The three mammalian GGAs mediate sorting at the TGN by binding to the DXXLL motif of cargo and recruiting clathrin for transport to early or late endosomes. It is unclear whether different GGAs (GGA1, 2, 3) are involved in different sorting events (15).

For the predominantly plasma membrane-localized CIC-2, two sorting motifs have been identified, a dileucine motif in the C-terminus that recruits AP-1 B for sorting to the basolateral membrane of epithelial cells (16) and a tyrosine-based motif in a cytoplasmic loop between helices D and E that mediates recycling between endosomes and the plasma membrane (17). Barttin, the β-subunit of CIC-K channels at the plasma membrane, carries an amino acid sequence (PQPPYVRL) likely to be involved in endocytosis (18). However, it is not clear whether the critical tyrosine belongs to a functional YXXΦ sorting motif or the sequence functions as a so-called PY motif for ubiquitylation-regulated internalization as in the case of the sodium channel ENaC (19). So far no 'conventional' AP- or GGA- binding motifs have been described for intracellular CLCs. CIC-3, which localizes to endocytic compartments (2,4,10,20-23) as well as to synaptic vesicles and synaptic-like microvesicles (2,3,24), has been shown to interact via its N-terminus with AP-1, AP-2 and clathrin (23). The interaction with clathrin was shown to be dependent on an acidic amino acid stretch with two dileucines whereas that with AP-2 did not require this stretch. Targeting of CIC-3 to synaptic vesicles and synaptic-like microvesicles has been reported to require AP-3 (3). Various motifs different from AP- or GGA-interaction sequences have been shown to be important for the subcellular localization of endo-lysosomal CLCs. A splice variant of CIC-3, CIC-3B, exhibits a PDZ-binding motif at its extreme carboxy-terminus (25), which recruits CIC-3B to the Golgi complex via an interaction with the PDZ domain-carrying Golgi protein GOPC (26). For CIC-4, which has mostly been reported to localize to endosomes (4,27), but also to the endoplasmic reticulum (ER) (28), an N-terminal amino acid stretch is reportedly involved in ER retention when expressed heterologously (28). The predominantly endosomal CIC-5 (4,27,29-32), a small portion of which is endogenously found also in the plasma membrane (30,33), bears a C-terminally located PY-motif (34). Although not required for *in vivo* CIC-5 function (33), studies in *Xenopus* oocytes and cultured opossum kidney cells revealed that ubiquitin ligases bind this motif and ubiquitylate CIC-5, stimulating its internalization from the cell surface (34,35). While endogenous CIC-6 localizes to late endosomes (36), heterologously expressed CIC-6 has been found on early and recycling endosomes (37). A basic amino acid stretch in CIC-6 seems to be involved in the recruitment of CIC-6 into detergent-resistant membranes, which may play a role in its subcellular localization (37). For CIC-7 or its Beta- subunit Ostm1 (38), no motifs responsible for their lysosomal localization (38-40) have been reported so far. CIC-7 is targeted to lysosomes in the absence of Ostm1, whereas Ostm1 requires CIC-7 to be exported from the ER (38).

This invention therefore relates to the systematic identification of the cytosolic sorting motifs of endosomal/lysosomal CLCs and their role in subcellular sorting. Through examination of motifs important in subcellular sorting, various sequences have been developed for the presentation of CIC-7 on the cell or plasma membrane and/or cell surface.

### SUMMARY OF THE INVENTION

In light of the prior art the technical problem underlying the present invention is to provide a CIC-7 protein that is presented on the cell surface and/or cell membrane, whereby the chloride transporter is physically accessible to test substances. The technical problem underlying the present invention could be further described as providing a cell-based assay system in which the CIC-7 protein is presented by the cell on the cell surface and/or cell membrane where it is accessible to substances being tested for CIC-7 binding and/or inhibition, thereby enabling a test system for interrogating CIC-7 activity.

This problem is solved by the features of the independent claims. Preferred embodiments of the present invention are provided by the dependent claims.

The invention therefore relates to CIC-7 protein that is presented on the cell surface and/or plasma membrane, characterised in that interaction between CIC-7 and the endosomal and/or lysosomal sorting machinery is inhibited and/or disrupted, whereby the endosomal and/or lysosomal sorting machinery is selected from the group comprising of one or more adaptor proteins (AP) and/or Golgi-localized, γ-ear containing, Arf-binding adaptor proteins (GGA).

According to the present invention the CIC-7 protein is characterised in that the amino acid sequences of one or more AP or GGA recognition motifs is mutated and/or deleted.

The invention therefore relates to a CIC-7 protein that is presented on the cell surface and/or plasma membrane, characterised in that interaction between CIC-7 and the endosomal and/or lysosomal sorting machinery is inhibited and/or disrupted, whereby the CIC-7 protein is selected from human, rat or mouse CIC-7 protein, wherein said CIC-7 protein comprises an N-terminal sequence according to SEQ ID NO: 19, 20 or 21, which is modified by a mutation in the following AP or GGA recognition motifs:
- In the human CIC-7 sequence the 19-EAAPLL-24 motif (SEQ ID NO: 2) and/or the 65-DDELL-69 motif (SEQ ID NO: 8),
- In the mouse and/or rat CIC-7 sequences the 19-EGAPLL-24 motif (SEQ ID NO: 4), the 32-EETPLL-37 motif (SEQ ID NO: 6) and/or the 63-DDELL-67 motif (SEQ ID NO: 10),
wherein said CIC-7 protein comprises at least a mutation in the 19-EAAPLL-24 motif (SEQ ID NO: 2) in human CIC-7 or 19-EGAPLL-24 motif (SEQ ID NO: 4) in rat or mouse CIC-7, wherein rat or mouse CIC-7 additionally comprises a mutation in the 32-EETPLL-37 motif (SEQ ID NO: 6).

The invention further relates to a CIC-7 protein that is presented on the cell surface and/or plasma membrane via a method that interferes with binding of CIC-7 to the endosomal/lysosomal sorting machinery. One such method relates to the functional disruption or sequestering of the endosomal and/or lysosomal sorting machinery, whereby the endosomal and/or lysosomal sorting machinery is preferably selected from the group comprising of one or more adaptor proteins (AP) and/or Golgi-localized, γ-ear containing, Arf-binding adaptor proteins (GGA). Such a disruption can also occur in addition to disruption of the interaction between CIC-7 and the endosomal and/or lysosomal sorting machinery

The CIC-7 protein of the invention is characterised in that the AP or GGA recognition motifs are selected from the group comprising: dileucine motifs, for example [DE]XXXL[LI] or DXXLL, or tyrosine-based motifs, for example YXXΦ (with Φ being a bulky, hydrophobic amino acid).

In a further embodiment the CIC-7 protein of the invention is characterised in that the mutation of AP or GGA recognition motifs comprise the CIC-7_{LL23/24AA}, CIC-7_{LL36/37AA}, CIC-7_{LL66/67AA} and/or CIC-7_{YF713/716AA} mutations, more preferably of the EGAPLL₂₄, EETPLL₃₇, DDELL₆₇, and/or Y₇₁₅PRF₇₁₈ motifs, especially the EGAPLL₂₄ and/or EETPLL₃₇ motifs.

In a further embodiment the CIC-7 protein of the invention is characterised in that the protein has another protein or epitope fused to it, resulting in cell surface targeting of the fusion protein.

In a further embodiment the CIC-7 protein of the invention is characterised in that the amino acid sequence comprises:
a) a sequence selected from the group comprising SEQ ID NO: 1, 3, 5, 7, 13, 14, 15, 16, 17 and 18.
b) a sequence having at least 80% sequence identity, preferably 90% sequence identity, to the sequence according to a).

A further aspect of the invention relates to a method for screening a test compound for activity in modulating chloride and/or proton transport via CIC-7 characterised in that
- the CIC-7 protein according to the invention is present on the cell surface and/or plasma membrane of a cell, whereby the protein is physically accessible to said test compound,
- said test compound is brought into contact with said cell, and
- proton transport and/or chloride transport, preferably via CIC-7, is measured,
- and optionally, that one or more adaptor proteins (AP) or Golgi-localized, γ-ear containing, Arf-binding adaptor proteins (GGA) are functionally disrupted and/or sequestered.

In further embodiments of the invention the modulation of chloride and/or proton transport can relate to either inhibition or blocking of chloride and/or proton transport or to enhancement or activation of chloride and/or proton transport.

In a preferred embodiment the method of the invention is characterised in that the method is a cell-based screening assay.

In another preferred embodiment the method of the invention is characterised in that the activity of the test compound in modulating chloride and/or proton transport via CIC-7 is measured by the change in transport of chloride ions across the cell membrane and/or the change in intracellular pH of a treated cell, preferably using pH-sensitive and/or chloride-sensitive detection agents, such as colouring agents.

The invention further relates to the method as described above, characterised in that
- Means for depolarising the cell are present, preferably that a ligand-gated sodium channel, preferably FaNaC, is co-expressed with the CIC-7 protein in the cell, or a sodium ionophore is present, and/or
- Ostm1 protein is co-expressed with the CIC-7 protein in the cell.

In another embodiment the method of the invention is characterised in that a ligand-gated sodium channel, preferably FaNaC, is co-expressed with the CIC-7 protein in the cell.

In another embodiment the method of the invention is characterised in that Ostm1 protein is co-expressed with the CIC-7 protein in the cell. The inhibition of the interaction between Ostm1 and CIC-7 can also be measured as a functional outcome of chloride transporter inhibition. The Ostm1 and CIC-7 interaction can occur between the mutant CIC-7 of the present invention with either endogenously or exogenously expressed Ostm 1.

Also disclosed is a method as described herein that further comprises formulating the identified compound that exhibits activity in modulating chloride and/or proton transport via CIC-7 in a pharmaceutically acceptable form.

Also disclosed is a method for the production of a pharmaceutical composition comprising the method as described herein and furthermore mixing the identified compound with a pharmaceutically acceptable carrier.

Also disclosed is a method as described above, whereby the pharmaceutical composition is suited and intended for application in the treatment of osteoporosis.

A further aspect of the invention relates to a nucleic acid molecule that encodes the CIC-7 protein of the present invention, as described above. A further aspect of the invention relates to a vector comprising the nucleic acid molecule as described above. A further aspect of the invention relates to a cell comprising the nucleic acid molecule and/or vector as described above.

A further aspect of the invention relates to a kit for carrying out the method as described herein, comprising the CIC-7 protein of the invention, the nucleic acid molecule and/or vector encoding the CIC-7 protein and/or the cell comprising such a nucleic acid molecule, in addition to means for measuring the change in transport of chloride ions across the cell membrane and/or the change in intracellular pH or chloride concentration of a treated cell, preferably using pH-sensitive and/or chloride-sensitive detection agents, such as colouring agents.

The invention further relates to the use of the CIC-7 as described herein in a cell-based screening method to identify CIC-7-modulatory activity of test compounds.

### DETAILED DESCRIPTION OF THE INVENTION

The method for screening according to the present invention is intended to identify an activity or the ability of a test compound or substance to modulate chloride and/or proton transport via CIC-7.

In a preferred embodiment the screening assay or method for screening a test compound is based on the change in transport of chloride ions and/or protons across the cell membrane and therefore the change in intracellular pH or chloride concentration of a treated cell, which can be detected and/or measured, preferably using pH-sensitive and/or chloride-sensitive detection agents, such as colouring agents.

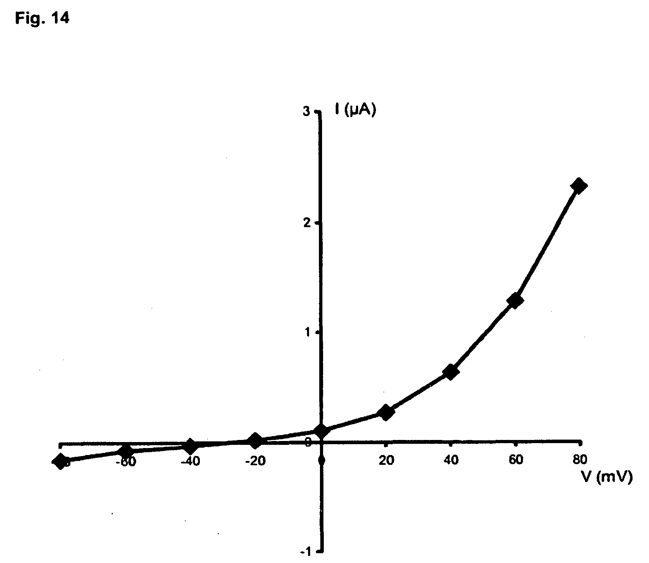

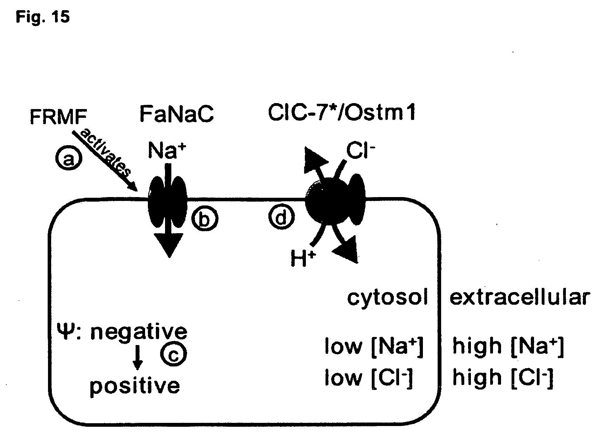

It is important to consider that CIC-7 is activated during intracellular depolarisation (similar to CIC-4 and CIC-5) (Fig. 14). In order to provide a system or method for screening potential CIC-7 modulators, FaNaC, a sodium channel from the snail Helix aspersa, can be co-expressed with the CIC-7 in the cell. The FaNaC channel localises on the plasma membrane and opens in response to addition of its ligand (the peptide FMRFamide). FaNaC and FMRFamide application is a useful tool for depolarisation of the cell. However, the use of FaNaC and FMRFamide is not a limiting aspect of the invention, whereby other methods, such as similar ligand-gated ion channels or Na-ionophores, may be used in the present invention. Due to the existing sodium gradient across the cell membrane (low sodium levels in the cell, high sodium levels outside the cell) depolarisation of the cell occurs. This in turn activates CIC-7 (Fig. 15).

A cell-based assay is therefore an intended aspect of the present invention, comprising cells which stably express the CIC-7 of the present invention, Ostm1and/or FaNaC. Functional analogues of Ostm1 and FaNaC (regarding their function in relation to CIC-7 and the assay of the present invention) are considered to be within the scope of the present invention.

Such a cell-based system could be applied in a small molecule screen for potential modulators of CIC-7 and/or the interaction between CIC-7 and Ostm1. Inhibitors of CIC-7 activity and/or the interaction between CIC-7 and Ostm1 would lead to a reduction in the change of pH (alkalinisation during chloride influx).

Screening for the ability of a compound of block one or more CIC-7 chloride transporters can however be performed by a multitude of techniques well known in the art. Examples of available screening methods are conventional electrophysiological methods such as patch-clamp techniques, or conventional spectroscopic methods such as FLIPR assay (Fluorescence Image Plate Reader; available from Molecular Devices), or VIPR (voltage ion probe reader, available from Aurora). These methods generally comprise monitoring the membrane potential of the chloride channel/transporter containing cell in order to identify changes in the membrane potential caused by the action of the compound. Other screening methods include ligand binding assays, CI-flux based assays and oocyte voltage clamp after expression of CIC cDNA in an appropriate heterologous system. Other methods for screening compounds for the ability to modulate the transport of chloride ions by CIC-7 have been proposed in WO02/059612 and in WO02/059356. These methods were based on the measurement of steady-state pH as a proxy for chloride ion transport in a cell or an organelle. The methods of the prior art can be applied in the screening method of the present invention. The present invention also represents an additional improvement over the known methods, as the steady-state pH remains unchanged in CIC-7 deficient cells.

The method of the present invention for screening a test-compound is particularly useful for application in high-throughput screening. One of the most difficult aspects of testing compounds for an activity against CIC-7 in the past was the intracellular localisation. Testing whether substances inhibited CIC-7 may have been possible for small numbers of isolated substances, although none of the methods of the prior art were capable of high throughput screening for many small molecules or other substances for their effect on CIC-7 chloride and/or proton transport. Because of the surface presentation of the CIC-7 protein according to the present invention a cell-based assay can be assembled that is suitable for screening large libraries of molecules, as are commonly known to those skilled in the art.

A useful dye for detecting changes in pH is BCECF, which exists also in a membrane-permeable form (BCECF-AM). This dye is processed by cytosolic esterases so that it will not be able to diffuse out through the plasma membrane. Instead of measuring intracellular pH, an additional method relates to measurement of cytosolic chloride using chloride-sensitive dyes, such as MEQ, or genetically encoded chloride sensors, such as 'clomeleon'. The former would be loaded into the cells before the assay, the latter transfected, ideally stably. Other pH-sensitive dyes can also be used in the present invention. Genetically encoded dyes or markers can also be used, for example GFP-expression, such as expression of GFP-variants, that may exhibit changes in signal via pH- or chloride-sensitivity, or for example when GFP or GFP variants are placed under a pH- or chloride-sensitive promoter. Other fluorescent dyes can also be used, as these are within the knowledge possessed by one skilled in the art.

The method for identifying or screening an agent or test compound with the inventive properties as described herein can be carried out using either manual or automated approaches. There are numerous experimental approaches provided throughout the disclosure of the present application, each of which are capable of being carried out manually, for example using small scale approaches with individual laboratory protocols, or in an automated fashion. The automated identification method can be carried out in a large scale, high-throughput manner, which is capable of testing chemical libraries, such as those containing small molecules and derivatives thereof, other molecules or RNAi libraries. The automation of such a method is in one embodiment preferred in that automated high throughput microscopy is applied, whereby mammalian cells exhibiting the CIC-7 proteins as described herein are treated with any number of compounds to be tested, thereby identifying the agents that exhibit the properties as described herein using the experimental criteria provided herein, especially those in the experimental examples provided herein.

Examples of the compounds of the present invention to be screened or identified include, but are not limited to: polypeptides, peptides, peptidomimetics or functional fragments thereof; nucleic acids or functional fragments thereof, or agents which modulate the function of CIC-7. Examples of small molecules include, but are not limited to, peptides, peptidomimetics (e.g., peptoids), amino acids, amino acid analogs, polynucleotides, polynucleotide analogs, nucleotides, nucleotide analogs, organic and inorganic compounds (including heterorganic and organometallic compounds).

The examples disclosed within the present invention utilise rat and human sequences.

When rat CIC-7 (rCIC-7) is used in the cell-based assay of the present invention, whereby the modified rCIC-7 protein is expressed in cells and is subsequently transported to the cell surface, the compounds identified through the cell-based assay as exhibiting rCIC-7-inhibitory activity may also exhibit such inhibitory activity against human CIC-7. Although slight differences exist between the CIC-7 protein sequences from different mammals regarding subcellular sorting properties, such differences do not imply that compounds which exhibit a CIC-7-inhibitory affect (identified through the assay of the present invention) against one species of CIC-7 (for example rat) do not exhibit the inhibitory effect against the CIC-7 proteins from other species (such as humans). Human CIC-7 protein as described herein can also be used in the screening assay according to the present invention.

The sequences of the present invention relate to mammalian sequences, whereby in some circumstances the exact amino acid position of a particular motif within the protein/open reading frame is different amongst the various mammals. Despite these differences the motifs as disclosed herein have the same or similar function to the corresponding motifs of other species, even if positioned in somewhat different places within the protein sequence.

The motifs provided herein can be modified in the specific way as disclosed herein, for example via double mutation of LL to AA. However, other mutations within these motifs are possible which would lead to the same or similar properties and these further mutations are within the range of capabilities of a skilled person and are therefore also encompassed in the present invention.

**Table 1. Description of sequences of the present invention (wt represents unmutated wild type sequence, aa represents amino acid, h represents human, r represents rat, m represents mouse).**

| **SEQ ID NO.** | **Description** | **Amino acid sequence** |
|---|---|---|
| SEQ ID NO: 1 | hCIC-7 LL23/24AA | EAAPAA |
| SEQ ID NO: 2 | hCIC-7 aa 19-24 wt | EAAPLL |
| SEQ ID NO: 3 | rCIC-7 or mCIC-7 LL23/24AA | EGAPAA |
| SEQ ID NO: 4 | rCIC-7 or mCIC-7 aa 19-24 wt | EGAPLL |
| SEQ ID NO: 5 | rCIC-7 or mCIC-7 LL36/37AA | EETPAA |
| SEQ ID NO: 6 | rCIC-7 or mCIC-7 aa 32-37 wt | EETPLL |
| SEQ ID NO: 7 | hCIC-7 LL68/69AA | DDEAA |
| SEQ ID NO: 8 | hCIC-7 aa 65-69 wt | DDELL |
| SEQ ID NO: 9 | rCIC-7 or mCIC-7 LL66/67AA | DDEAA |
| SEQ ID NO: 10 | rCIC-7 or mCIC-7 aa 63-67 wt | DDELL |
| SEQ ID NO: 11 | rCIC-7 & mCIC-7: YF713/716AA, hCIC-7: YF715/718AA | APRA |
| SEQ ID NO: 12 | rCIC-7 & mCIC-7: aa 713-716 wt, hCIC-7: aa 715-718 wt | YPRF |
| SEQ ID NO: 13 | aa 1 -126 human CIC-7 mutant (mutated AP or GGA recognition motifs are underlined). | |
| SEQ ID NO: 14 | aa 1 -124 rat CIC-7 mutant (mutated AP or GGA recognition motifs are underlined). | |
| SEQ ID NO: 15 | aa 1 -124 mouse CIC-7 mutant (mutated AP or GGA recognition motifs are underlined). | |
| | | |
| SEQ ID NO: 16 | aa 1 -126 human CIC-7 mutant (mutated AP or GGA recognition motifs are underlined). | |
| SEQ ID NO: 17 | aa 1 -124 rat CIC-7 mutant (mutated AP or GGA recognition motifs are underlined). | |
| SEQ ID NO: 18 | aa 1 -124 mouse CIC-7 mutant (mutated AP or GGA recognition motifs are underlined). | |
| SEQ ID NO: 19 | aa 1 -126 human CIC-7 wt | |
| SEQ ID NO: 20 | aa 1 -124 rat CIC-7 wt | |
| SEQ ID NO: 21 | aa 1 -124 mouse CIC-7 wt | |

The present invention is characterised by N-terminal sequences of the CIC-7 protein, which exhibit mutations in the following specific AP or GGA recognition motifs:
- In the human CIC-7 sequence the 19-EAAPLL-24 motif (SEQ ID NO: 2) and/or the 65-DDELL-69 motif (SEQ ID NO: 8).
- In the mouse and/or rat CIC-7 sequences the 19-EGAPLL-24 motif (SEQ ID NO: 4), the CIC-7 32-EETPLL-37 motif (SEQ ID NO: 6) and/or the 63-DDELL-67 motif (SEQ ID NO: 10).

Preferred embodiments of the present invention encompass N-terminal sequences of the CIC-7 protein, which exhibit the following specific mutations:
- In the human sequence the CIC-7 LL23/24AA mutation (SEQ ID NO: 1, SEQ ID NO: 13, SEQ ID NO: 16).
- In the human sequence the CIC-7 LL68/69AA mutation (SEQ ID NO: 7, SEQ ID NO: 13).
- In the mouse and/or rat sequences the CIC-7 LL23/24AA (SEQ ID NO: 3, SEQ ID NO: 14, SEQ ID NO: 15, SEQ ID NO: 17, SEQ ID NO: 18,).
- In the mouse and/or rat sequences the CIC-7 LL36/37AA mutations (SEQ ID NO: 5, SEQ ID NO: 14, SEQ ID NO: 15, SEQ ID NO: 17, SEQ ID NO: 18).

The wt sequences are provided in table 1 as SEQ ID NOs: 2, 4, 6, 8, 10, 12, 19, 20 and 21. Particularly preferred sequences are those according to SEQ ID NOs: 13-18.

Also encompassed within the scope of the present invention are mutants of the CIC-7 protein that result in faster activation than the wild-type protein. These faster mutants encompass mutations that can be used separately or additionally to the mutations described in the sequences provided in Table 1. Various mutations that lead to faster channelling are possible, and in a preferred embodiment are combined with above-mentioned mutants that lead to cell-surface localisation. Such mutants are suitable for application in the cell-based assay as described herein. One example of a mutation that leads to faster channelling is the R762Q mutation. This mutation relates to the human amino acid sequence numbering.

The desired effect of mis-targeting CIC-7 to the plasmas membrane can be achieved via various mutations to AP or GGA recognition motifs. Such mutations are within the reach of one skilled in the art and are therefore encompassed by the present invention. Further mutations that can be used are for example the replacement of the double leucine motif with a double glycine. Other amino acids of the AP or GGA recognition motifs, alternatively or in addition to the double leucine motif, may also be mutated to achieve the desired properties of the CIC-7 protein of the present invention.

Sequence variants of the claimed CIC-7 proteins that are presented on the cell surface and/or plasma membrane, said variants comprising a sequence having at least 80% sequence identity to a sequence selected from the group comprising SEQ ID NO: 1, 3, 5, 7, 13, 14, 15, 16, 17 and 18 and that maintain the said properties of the invention, are also included in the scope of the invention. Protein modifications which occur through substitutions are also included within the scope of the invention. Substitutions as defined herein are modifications made to the nucleic acid or amino acid sequence of the protein, producing a protein which contains a different amino acid sequence than the primary protein without significantly altering the function of the protein. Like additions, substitutions may be natural or artificial. It is well known in the art that amino acid substitutions may be made without significantly altering the protein's function. This is particularly true when the modification relates to a "conservative" amino acid substitution, which is the substitution of one amino acid for another of similar properties. Such "conserved" amino acids can be natural or synthetic amino acids which because of size, charge, polarity and conformation can be substituted without significantly affecting the structure and function of the protein. Frequently, many amino acids may be substituted by conservative amino acids without deleteriously affecting the protein's function.

In general, the non-polar amino acids Gly, Ala, Val, Ile and Leu; the non-polar aromatic amino acids Phe, Trp and Tyr; the neutral polar amino acids Ser, Thr, Cys, Gln, Asn and Met; the positively charged amino acids Lys, Arg and His; the negatively charged amino acids Asp and Glu, represent groups of conservative amino acids. This list is not exhaustive. For example, it is well known that Ala, Gly, Ser and sometimes Cys can substitute for each other even though they belong to different groups. Conservative amino acid substitutions are not limited to naturally occurring amino acids, but also include synthetic amino acids. Commonly used synthetic amino acids are omega amino acids of various chain lengths and cyclohexyl alanine which are neutral non-polar analogs; citulline and methionine sulfoxide which are neutral non-polar analogs, phenylglycine which is an aromatic neutral analog; cysteic acid which is a positively charged analog and ornithine which is a negatively charged amino acid analog. Like the naturally occurring amino acids, this list is not exhaustive, but merely exemplary of the substitutions that are well known in the art.

Protein modifications or mutations may also occur through deletions. Deletions as defined herein are modifications made to the nucleic acid or amino acid sequence of the protein which produce a protein containing at least one amino acid less than the primary amino acid sequence of the protein, without significantly altering the function of the toxin. Like additions, naturally occurring deletions within the coding region of the protein usually severely impair the function of the protein, while deletions in the 5' and 3' untranslated region do not affect the function of the protein.

The active substances identified by the screening method of the present invention are intended for application in the treatment of osteoporosis. Osteoporosis is a progressive disease characterized by loss of bone calcification and mass and subsequent risk of bone fractures, resulting characteristically from the decrease in naturally occurring estrogen levels in post menopausal women. As many as 24 million post-menopausal women in the U.S. and an even higher number throughout Europe and Japan are affected. Osteoporosis accounts for as many as 1 million bone fractures per year in the U.S., with as many as 4 million throughout the developed countries. These fractures are responsible for substantially greater morbidity and mortality than the fractures suffered by younger patients.

The test compounds with identified CIC-7-inhibitory activity can be used, for example, in regenerating cortical and cancellous bone which has been lost as a result of post-menopausal osteoporosis, male idiopathic osteoporosis, congenital osteoporosis and other bone resorption diseases.

As discussed herein, the active substances identified via the method of the present invention may be used in a pharmaceutical composition or medicament when combined with a pharmaceutically acceptable carrier. The term "pharmaceutically acceptable" means a non-toxic material that does not interfere with the effectiveness of the biological activity of the active ingredient(s). The characteristics of the carrier will depend on the route of administration. Such a composition may contain, in addition to the active substance and carrier, diluents, fillers, salts, buffers, stabilizers, solubilizers, and other materials well known in the art. Formulation of pharmaceutically-acceptable excipients and carrier solutions is well-known to those of skill in the art, as is the development of suitable dosing and treatment regimens for using the particular compositions described herein in a variety of treatment regimens, including e.g., oral, parenteral, intravenous, intranasal, and intra-muscular administration and formulation.

The medicament, otherwise known as a pharmaceutical composition, containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavouring agents, colouring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastointestinal tract and thereby provide a sustained action over a longer period. For example, a time delay material such as glyceryl monostearate or glyceryl distearate may be employed. They may also be coated.

The present disclosure refers to a pharmaceutical composition for topical application, oral ingestion, inhalation, or cutaneous, subcutaneous, or intravenous injection. Topical application is one preferred embodiment, especially via a cream or gel. Topical application forms relate to gels, creams, suppositories, or films. The pharmaceutical composition of the invention may be in the form of a liposome in which the active substance of the invention is combined, in addition to other pharmaceutically acceptable carriers, with amphipathic agents such as lipids which exist in aggregated form as micelles, insoluble monolayers, liquid crystals, or lamellar layers which are in aqueous solution. Suitable lipids for liposomal formulation include, without limitation, monoglycerides, diglycerides, sulfatides, lysolecithin, phospholipids, saponin, bile acids, and the like. Preparation of such liposomal formulations is within the level of skill in the art.

The term "therapeutically effective amount" means the total amount of each active component of the pharmaceutical composition or method that is sufficient to show a meaningful patient benefit. The amount of active substance in the pharmaceutical composition of the present invention will depend upon the nature and severity of the condition being treated, and on the nature of prior treatments which the patient has undergone. Larger doses may be administered until the optimal therapeutic effect is obtained for the patient, and at that point the dosage is not increased further.

The abbreviations used are: aa, amino acid; AP, adaptor protein complex; CBS, cystathione Beta-synthetase; CLC, a gene family of CI-channels and transporters first identified by the cloning of CIC-0 from *Torpedo marmorata;* CT, carboxy-terminal domain; ER, endoplasmic reticulum; GFP, green fluorescent protein; GGA, Golgi-localized, γ-ear containing, Arf-binding (adaptor protein); GST, glutathion-S-transferase; HEPES, 4-(2-hydroxyethyl)-1-piperazineethanesulfonic acid; HRP, horseradish peroxidase; LAMP-1, lysosome-associated membrane protein-1; NT, amino-terminal domain; ORF, open reading frame; PCR, polymerase chain reaction; TfR, transferrin receptor; TGN, trans-Golgi network; WT, wildtype.

### FIGURES

- Figure 1.: Interaction between cytosolic CLC or Ostm1 domains with the sorting machinery.
- Figure 2.: Identification of adaptor protein binding sites.
- Figure 3.: Cell surface expression of CIC-5 mutants.
- Figure 4.: Subcellular sorting of CIC-6.
- Figure 5.: Lysosomal sorting determined by the CIC-7 amino-terminus.
- Figure 6.: Motifs responsible for the sorting of full-length CIC-7.
- Figure 7.: CIC-7-dependent transport of Ostm1.
- Figure 8.: Ponceau stainings of eluates from pull-down experiments of Fig. 1.
- Figure 9.: Further pull-down with the CIC-6 carboxy-terminal domain and ponceau stainings of eluates from pull-down experiments of Fig. 2.
- Figure 10.: Immunostaining of HeLa cells transiently expressing CIC-6, the chimera 6-0-6 or CIC-0.
- Figure 11.: Comparison of the amino-terminal regions of CIC-7 from human, mouse, rat, chicken and frog.
- Figure 12.: Subcellular localisation of 7-0-0 chimeras with further mutations.
- Figure 13.: Subcellular localisation of CLC-7 with further mutations.
- Figure 14.: Depolarisation-induced currents in oocytes expressing mutated CIC-7.
- Figure 15.: Model of cell-based assay.
- Figure 16.: Basic characterization of CIC-7PM /Ostm1 in Xenopus oocytes.
- Figure 17.: Modulation of CIC-7/Ostm1 by anions, protons and temperature.

A detailed description of the figures is provided below.

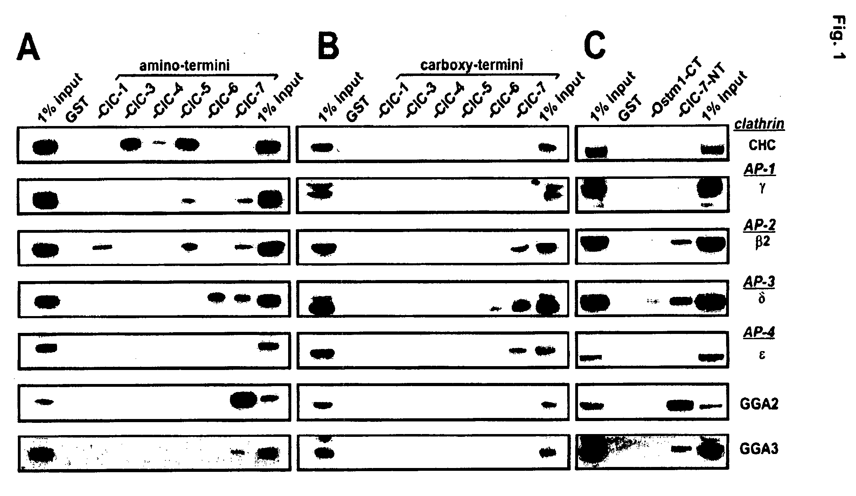

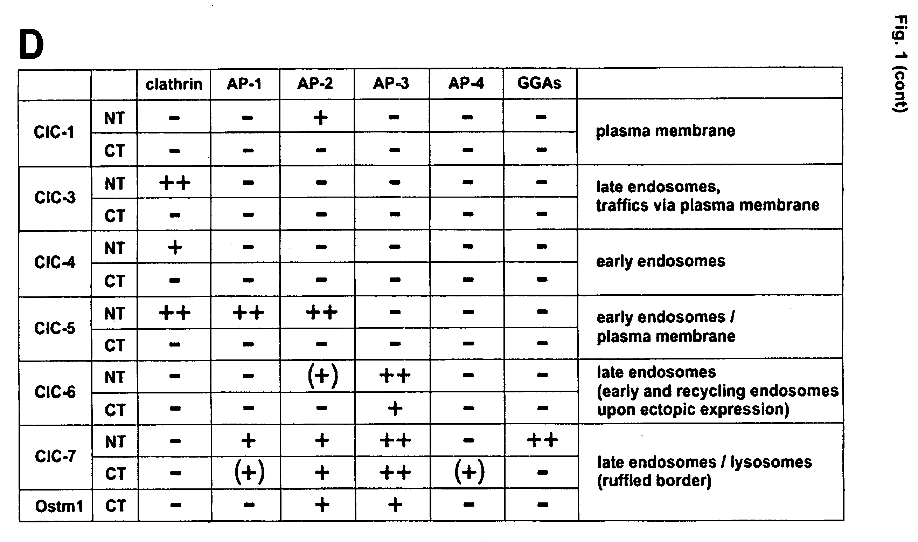

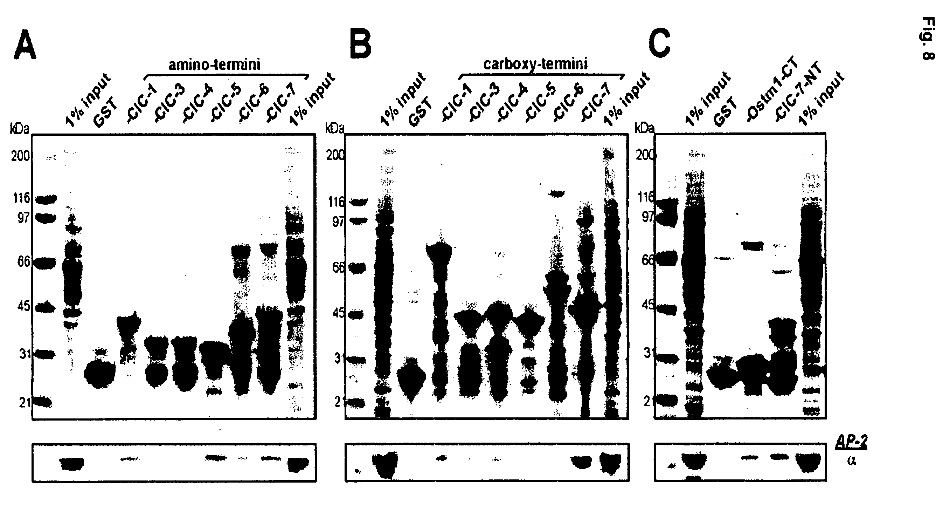

Fig. 1. Interaction between cytosolic CLC or Ostm1 domains with the sorting machinery. *A*-*C*, Western blots of eluates from GST pull-down experiments from HeLa cell lysates with the N-terminal (A) and C-terminal (*B*) cytosolic domains of CIC-1 and CIC-3 through -7 and the C-terminal domain of Ostm1 (C). 1% input is shown as control. Clathrin and adaptor proteins were detected with antibodies against clathrin heavy chain (CHC), γ-adaptin (for AP-1), Beta2-adaptin (for AP-2), Delta-adaptin (for AP-3), ε-adaptin (for AP- 4), GGA2 and GGA3. Ponceau stainings from these pull-downs showing similar amounts of bait proteins and an immunoblot against α-adaptin (AP-2) are given in Fig. 8. *D*, Summary of various pulldown experiments from HeLa cell, mouse brain and kidney lysate as shown in (*A-C*). -, no binding detected; (+), not detected in all experiments; +, binding always detected; ++, always strongly detected. The right column summarizes the published subcellular localization of the respective CLC.

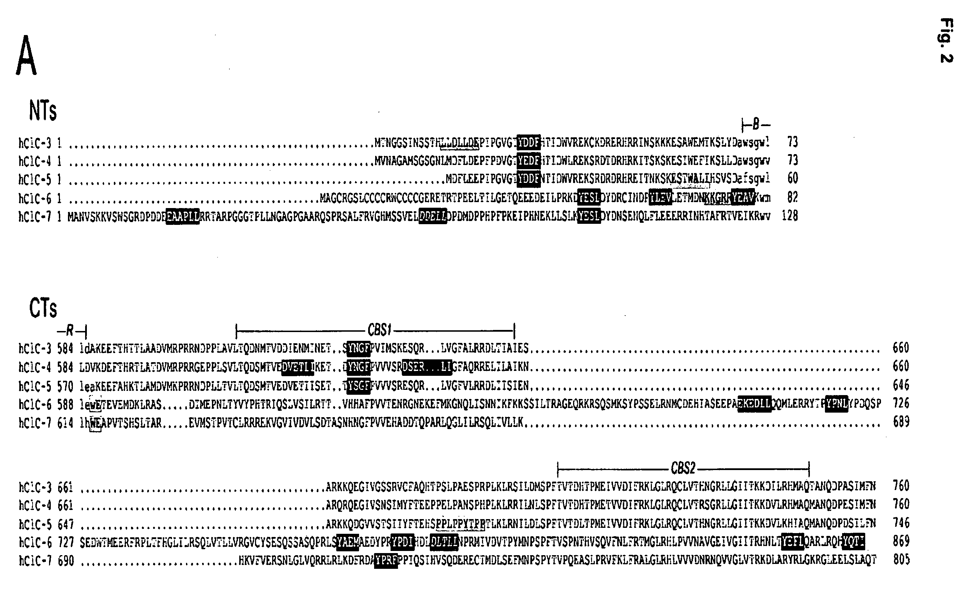

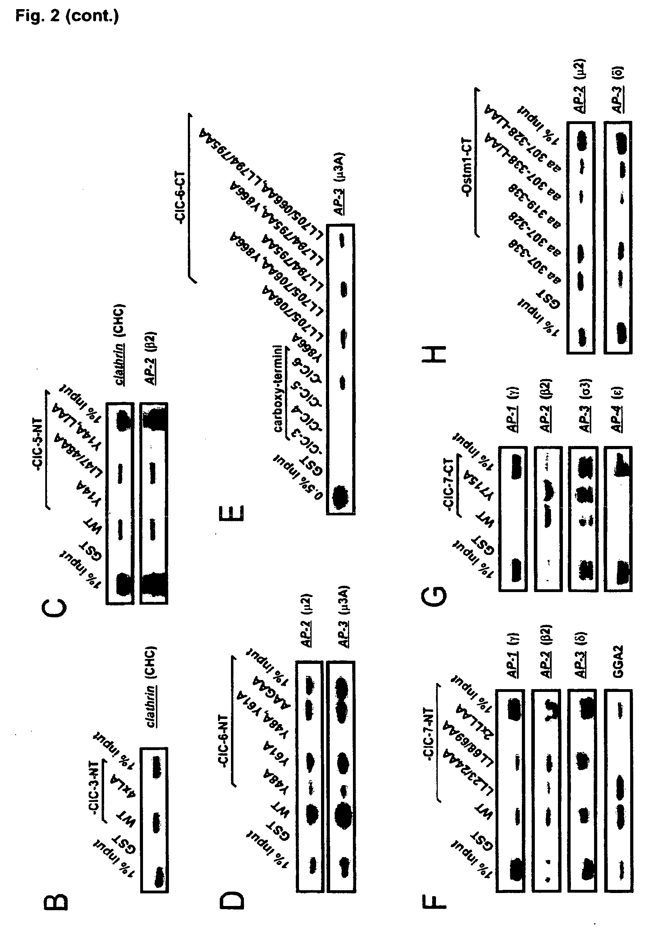

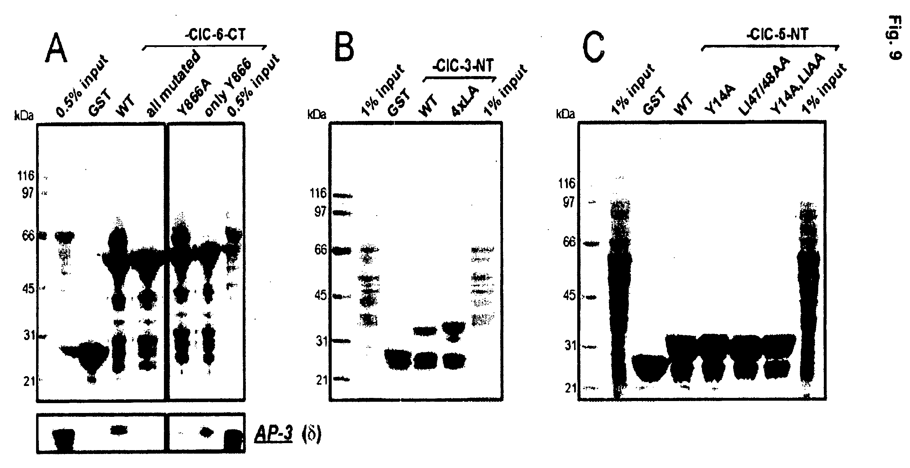

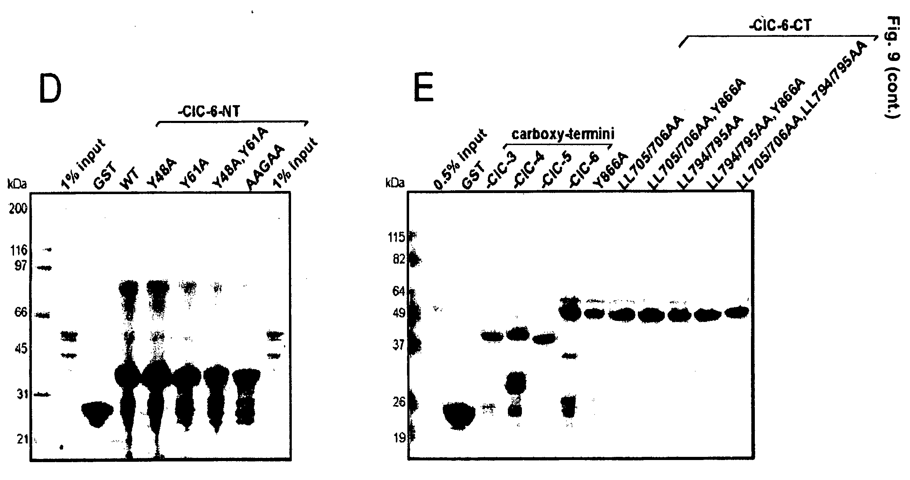

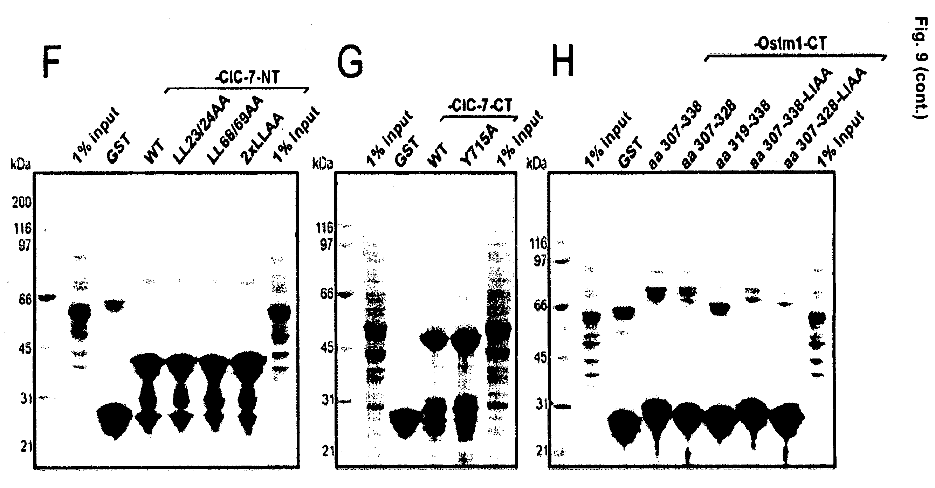

Fig. 2. Identification of adaptor protein binding sites. A, comparison of the amino- and carboxy-terminal regions of human CIC-3 through -7. Residues shown in lower case were not included in the GST fusion proteins. The two conserved CBS domains and the beginning of helix B and end of helix R are indicated. Potential AP-binding ([DE]XXXL[LI] and YXXΦ) and GGA-binding (DXXLL, italics) sorting motifs are highlighted as white on black background. Gray boxes indicate an N-terminal leucine-rich stretch in CIC-3 (23), the N-terminal ESTWALI₄₈ sequence and the PY motif in CIC-5 (34), a basic amino acid stretch in the N-terminus of CIC-6 (37), and a putative unconventional sorting motif (WE) (49) in the Ctermini of CIC-6 and -7, respectively. *B-H*, Western blots of eluates from pull-down experiments exploring the binding of interactors identified in Fig. 1 to the respective fusion proteins, either 'WT' or mutants in candidate binding motifs. Eluates from GST columns and 1% (0.5% in E) input shown as controls. For Ponceau staining showing similar amounts of bait protein see Fig. 9. *B*, clathrin binding to the CIC-3 N-terminus is almost abolished when all four leucines of the L₁₃LDLL sequence are changed to alanines (4xLA). C, binding of clathrin and Beta2-adaptin (AP-2) in pull-down from mouse kidney lysate to the N-terminus of CIC-5, a mutant in which Leu₄₇Ile₄₈ of the ESTWALI₄₈ sequence was replaced by alanines (LI47/48AA), and to mutants in which Y14 of the Y₁₄DDF motif was changed to alanine either alone (Y14A) or in addition to the LI47/48AA mutation (Y14A/LIAA). *D*, Binding of µ2 (AP-2) and µ3A (AP-3) from mouse brain lysate to the N-terminus of CIC-6, either WT or mutants in the Y₄₈ESL and Y₆₁LEV motifs (Y48A and Y61A), the double mutant (Y48A,Y61A), and the AAGAA mutant in the previously described K₇₁KGRR motif (37). *E*, µ3A (AP-3) binding in pull-down from HeLa cell extract with the C-termini of CIC-3, -4 and -5, and the CIC-6 C-terminus, either WT, the Y866A mutant in the Y₈₆₆QTI motif at the extreme C-terminus, a mutant (LL705/706AA) replacing both leucines of the EKEDLL₇₀₆ motif for AP binding by alanines, a similar mutant (LL794/795AA) in the DLTLL₇₉₅ consensus sequence for GGA-binding, and fusion proteins containing these mutations in various combinations. *F*, pull-down from HeLa cell extract with the wildtype N-terminus of human CIC- 7 (WT), a mutant with both leucines of EAAPLL₂₄ changed to alanine (LL23/24AA), a similar mutant of the DDELL₆₉ sequence (LL68/69AA), and a mutant combining these mutations (2xLLAA). G, pull-down from mouse brain lysate with the C-terminus of hCIC-7, either WT or a mutant (Y715A) in the Y₇₁₅PRF motif. *H*, pull-down from HeLa cell extract with the C-terminus of Ostm1 (aa 307-338) and various fragments thereof: a membrane-proximal fragment (aa 307-328), an extreme C-terminal fragment (aa 319-338), and mutants changing Leu₃₁₈Ile₃₁₉ to dialanine either in the complete C-terminus (aa 307-338- LIAA) or in the membrane-proximal fragment (aa 307-328-LIAA).

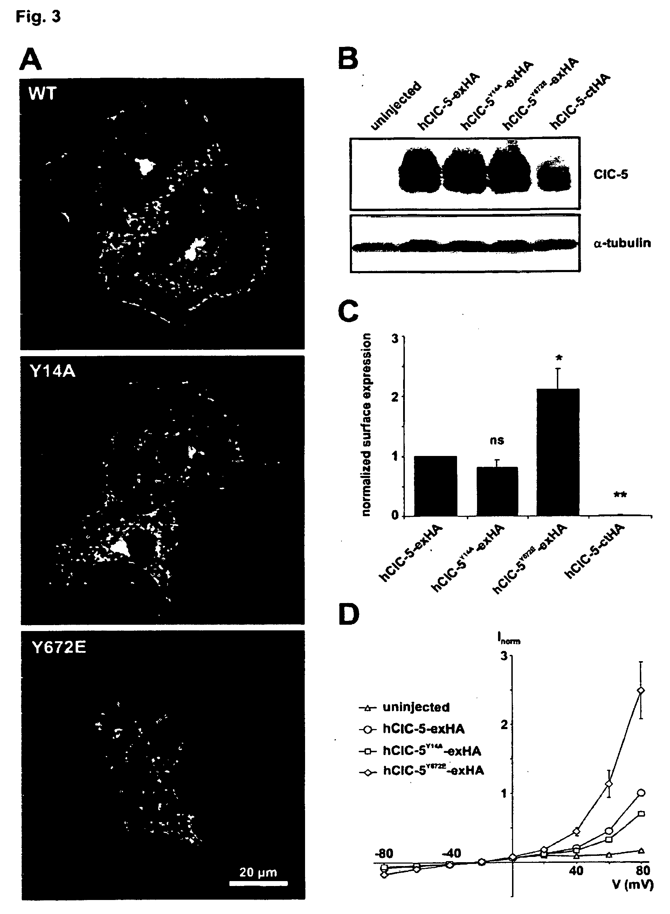

Fig. 3. Cell surface expression of CIC-5 mutants. A, subcellular localization in transfected CHO cells of either WT CIC-5 (top), a CIC-5 mutant carrying the Y14A mutation in the Y₁₄DDF motif involved in binding AP2 and clathrin, or the Y672E mutation in the PY-motif. Detection used the anti-CIC-5 antibody in immunofluorescence. All constructs show prominent vesicular staining and plasma membrane localization. B, immunoblot against CIC-5 of lysates from *Xenopus* oocytes injected with hCIC-5 WT, Y14A, Y672E (all with an extra-cytosolic HA-tag) or with hCIC-5 carrying an HA-tag at the C-terminus (hCIC-5-ctHA) showed similar expression levels of the respective protein. Immunoblot for tubulin served as loading control. C, surface expression of HA epitope-tagged hCIC-5 (hCIC-5-exHA) and its mutants Y14A and Y672E in *Xenopus* oocytes determined in a chemiluminescence assay. hCIC-5 carrying an intra-cytosolic, C-terminal HA-tag (hCIC-5-ctHA) served as control. Values are mean luminescence (± SEM) normalized to hCIC-5_{exHA} in 5 experiments (9-30 oocytes per construct each). ns, non-significant; *, p < 0.05; **, p < 0.001 compared to hCIC-5-exHA by Student's *t*-test. *D*, steady-state currents measured by two-electrode voltage-clamp of *Xenopus* oocytes injected with cRNA encoding hCIC-5 wildtype (WT), Y14A or Y672E (all with an extracytosolic HA tag), or uninjected oocytes as control. Values are mean current ± SEM normalized to WT at +80 mV (1.56 ± 0.24 µA) from 4 batches of oocytes (2-5 uninjected oocytes and 6-10 oocytes per construct each). Error bars are often hidden under the symbol for small SEMs.

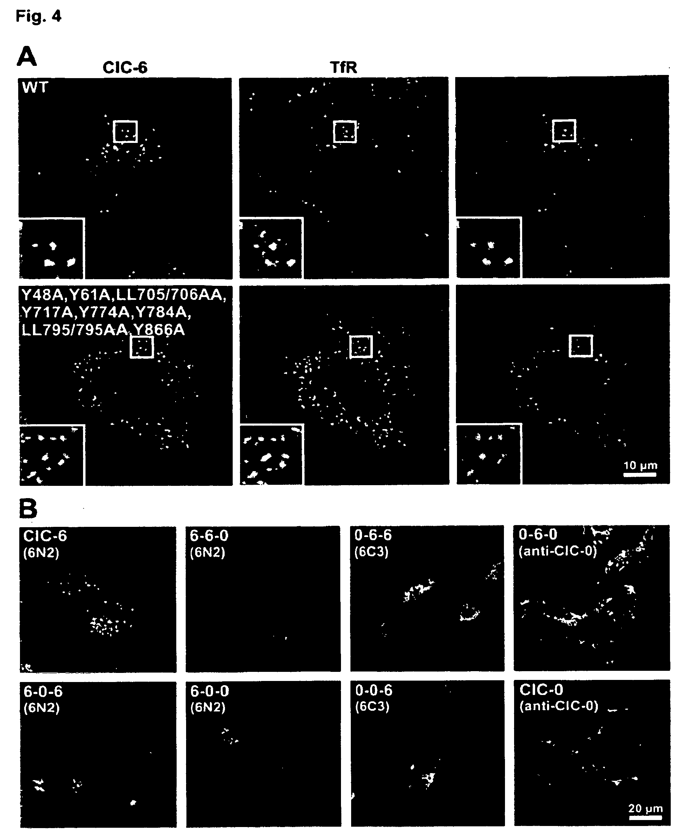

Fig. 4. Subcellular sorting of CIC-6. A, immunostaining of HeLa cells transiently transfected with WT CIC-6 (top panel) or CIC-6_{Y48A,Y61A,LL705/706AA,Y717A,Y774A,Y784A,LL795/796AA,Y866A} (below) for CIC-6 (green in merge) and the transferrin receptor (TfR, red in merge). Both CIC-6 proteins colocalize strongly with the TfR (yellow). B, subcellular localization of chimeras between CIC-6 and CIC-0 in transiently transfected HeLa cells. In chimeras the N-terminus (first number of the name), the transmembrane region (second number) and the C-terminal domain (third number) carry the respective parts of CIC-6 (6) or CIC-0 (0). Immunostaining used antibodies directed against the N-terminus of CIC-6 (6N2) or the C-terminus of CIC-6 (6C3) or of CIC-0 (antibodies indicated in brackets).

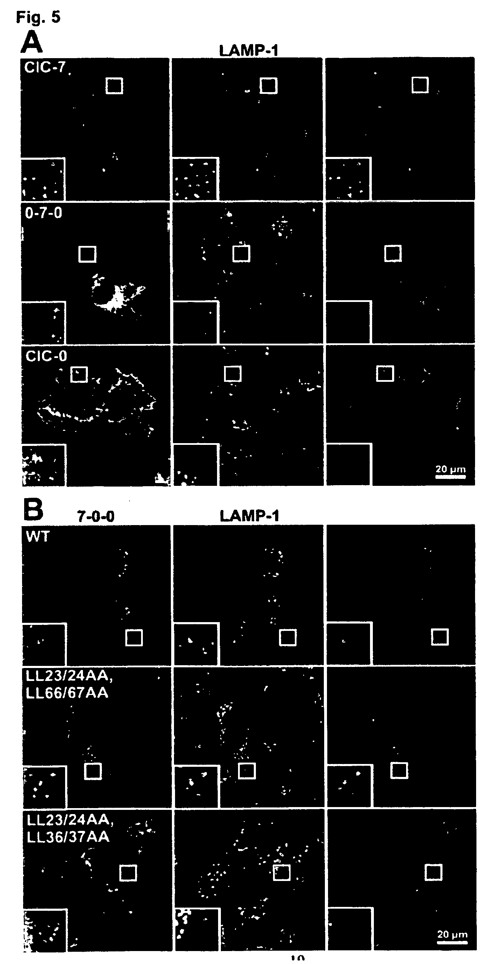

Fig. 5. Lysosomal sorting determined by the CIC-7 amino-terminus. A, subcellular localization of rCIC-7 (top), a chimera of rCIC-7 with N- and C-terminal domains replaced by those of CIC-0 (0-7-0, below), and WT CIC-0 (bottom) after transient transfection of HeLa cells, in comparison to LAMP-1 as marker for late endosomes and lysosomes. CIC-7 colocalizes with LAMP-1, whereas CIC-0 shows plasma membrane expression. The 0-7-0 chimera shows weak plasma membrane expression in addition to strong ER-like staining. *B*, sorting determinants in the CIC-7 N-terminus investigated in HeLa cells transfected with a chimeric protein (7-0-0) in which the N-terminus of the plasma membrane channel CIC-0 was replaced by that of rCIC-7 (top panel) or by CIC-7 N-termini carrying two combinations of mutations in the EGAPLL₂₄, EETPLL₃₇, and DDELL₆₇ dileucine motifs (EETPLL₃₇ present in rat, but not humans). The N-terminus of CIC-7 sufficed to target CIC-0 to lysosomes, and combined disruption of the first two motifs in 7_{LL23/24AA,LL36/37AA}-0-0 resulted in cell surface localization.

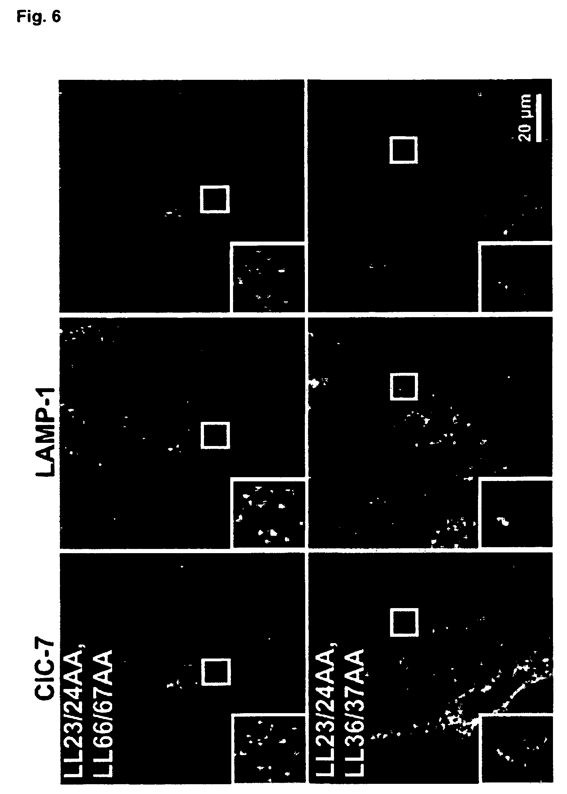

Fig. 6. Motifs responsible for the sorting of full-length CIC-7. HeLa cells fixed 28 h after transient transfection of full-length rCIC-7 carrying two combinations of mutations in the EGAPLL₂₄, EETPLL₃₇, and DDELL₆₇ dileucine motifs were immunostained with antibodies against CIC-7 (green in merge) and the late endosomal/lysosomal marker LAMP-1 (red in merge). While rCIC-7_{LL23/24AA,LL66/67AA} (upper panel) almost completely colocalized with LAMP-1 (yellow), rCIC-7_{LL23/24AA,LL36/37AA}-transfected cells (lower panel) displayed CIC-7 staining at the plasma membrane in addition to colocalization with LAMP- 1.

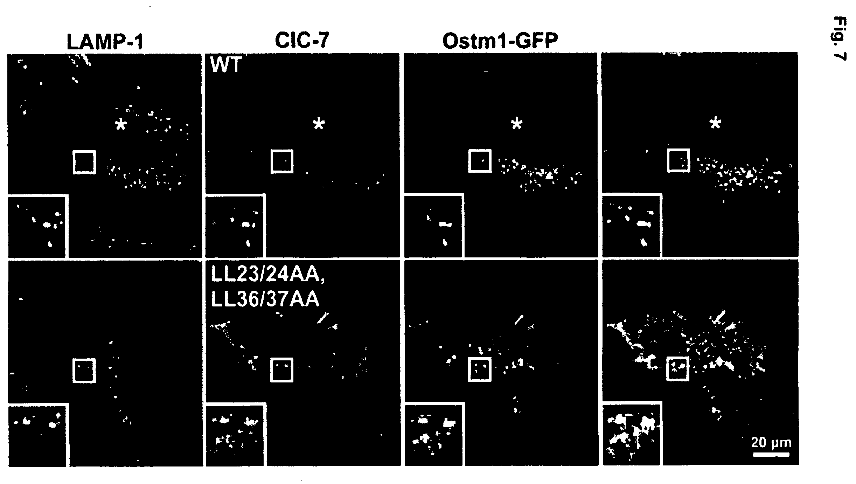

Fig. 7. CIC-7-dependent transport of Ostm1. Subcellular localization of LAMP-1 (magenta in merge), CIC-7 (yellow in merge) and Ostm1 (cyan in merge) in HeLa cells transiently co-transfected with Ostm1- GFP and WT rCIC-7 (upper panel) or the rCIC-7_{LL23/24AA,LL36/37AA} mutant that shows partial cell surface expression (lower panel). While Ostm1-GFP stains an ER-like pattern in cells that do not overexpress CIC-7 strongly (asterisk), it colocalizes with CIC-7 to LAMP-1-positive late endosomes/lysosomes in rCIC-7-overexpressing cells, and with endosomes/lysosomes as well as the plasma membrane in cells expressing rCIC-7_{LL23/24AA, LL36/37AA}.

Fig. 8. Ponceau stainings of eluates from pull-down experiments of Fig. 1 revealed that similar amounts of GST fusion proteins were used as bait. An additional immunoblot against the α-adaptin subunit of AP-2 is shown as an example of blots for further AP subunits that gave the same binding pattern as those for other subunits of the same AP complex as shown in Fig. 1A-C.

Fig. 9. A, eluate of pull-down from HeLa cell extract with GST fusion, proteins of the CIC-6 carboxy-terminal domain (as in Fig. 2E but with a different subset of mutants), Ponceau-stained for proteins (top) and immunoblotted for the AP-3 subunit δ-adaptin. Mutating tyrosine Y866 alone to alanine (Y866A) diminished binding of δ-adaptin as strongly as the combined mutation of the tyrosines and dileucines, respectively, Leu₇₀₅Leu₇₀₆, Tyr_{717,} Tyr₇₇₄, Tyr₇₈₄, Leu₇₉₄Leu₇₉₅ and Tyr₈₆₆, to alanines (all mutated). Mutating all of these except for Tyr₈₆₆ (only Y₈₆₆) did not strongly affect binding of AP-3 compared to wildtype (WT). *B-H,* Ponceau stainings of the corresponding immunoblots shown in Fig. 2BH revealed that similar amounts of WT constructs and point mutants of the GST fusion proteins were used as bait in the respective pull-down experiments.

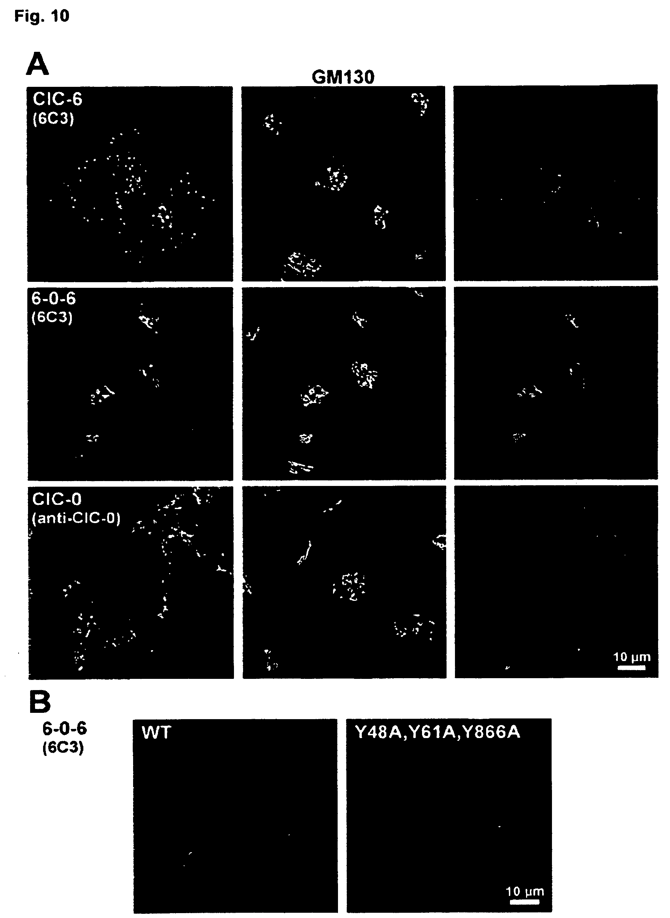

Fig. 10. A, immunostaining of HeLa cells transiently expressing CIC-6, the chimera 6-0-6 (amino-and carboxy-terminal domains of CIC-6, transmembrane region of CIC-0) or CIC-0 with antibodies directed against the cis-Golgi marker GM130 (red in merge) and the C-terminus of either CIC-6 (CIC-6 and 6-0-6) or CIC-0 (CIC-0) (green in merge). B, immunostaining with an anti-CIC-6 antibody of HeLa cells expressing either the chimera 6-0-6 or the one in which tyrosines Tyr₄₈, Tyr₆₁ and Tyr₈₆₆ of the fulllength CIC-6 sequence were mutated to alanines showed that the perinuclear localization of 6-0-6 is not altered by those mutations.

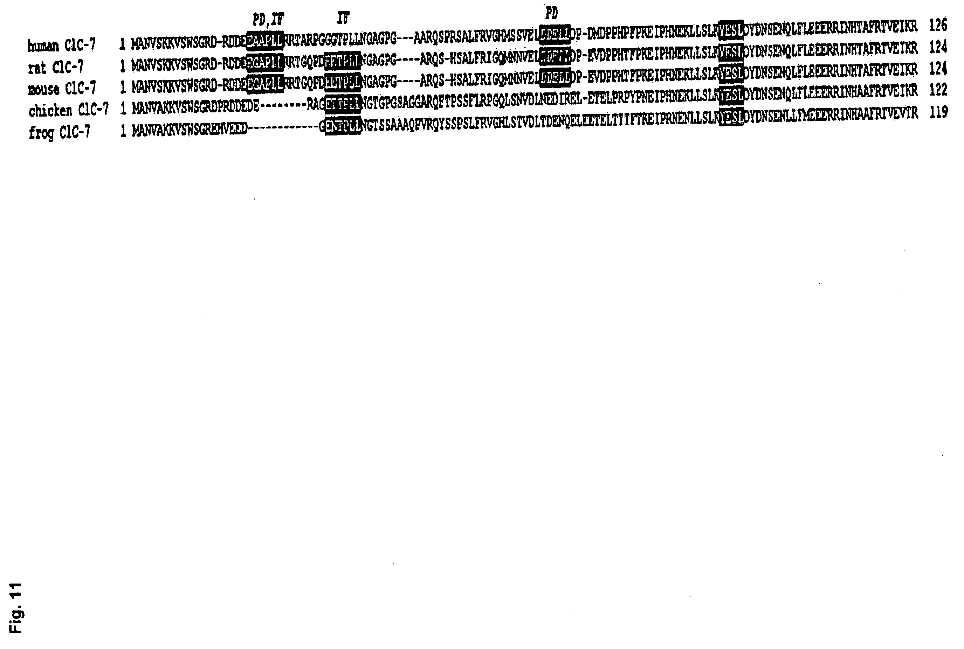

Fig. 11. Comparison of the amino-terminal regions of CIC-7 from human, mouse, rat, chicken and frog. Potential AP-binding ([DE]XXXL[LI] and YXXΦ) and GGA-binding (DXXLL) sorting motifs are highlighted as white on black background (the latter in italics). *PD* and *IF* above a motif indicate that we showed its involvement in AP complex or GGA protein binding in our pull-down experiments with human CIC-7 and in lysosomal sorting in our subcellular localization studies with rat CIC-7, respectively.

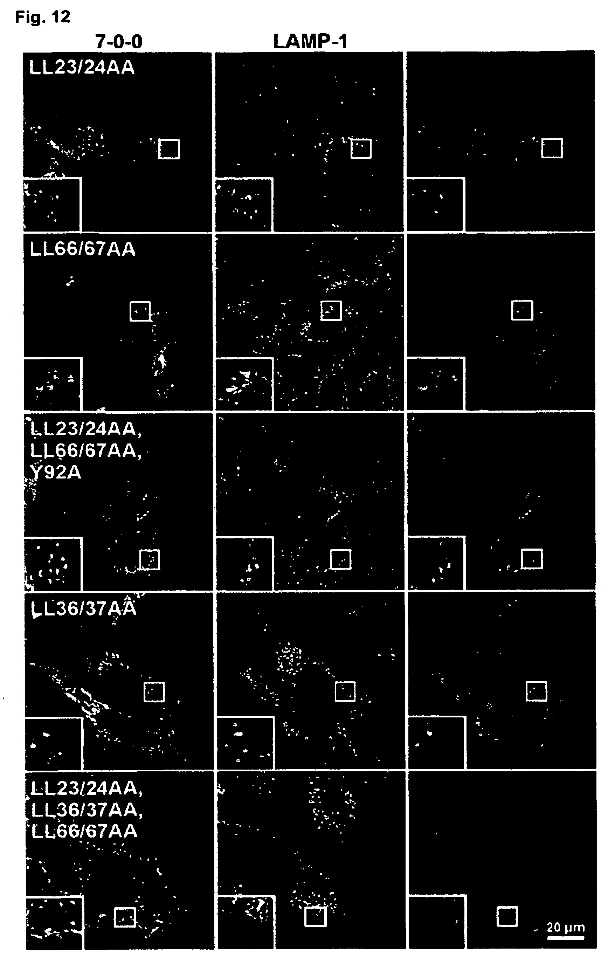

Fig. 12. Immunocytochemistry showing subcellular localization of transfected 7-0-0 chimeras with further mutations in the CIC-7 N-terminus compared to LAMP-1 staining in HeLa cells. The amino-terminal region of CIC-0 replaced by that of rCIC-7, as in Fig. 5B, but carrying different combinations of point mutations. Costaining with antibodies against the carboxy-terminal region of CIC-0 (green in merge) and the late endosomal/lysosomal marker protein LAMP-1 (red in merge) revealed that 7_{LL23/24AA}-0-0, 7_{LL66/67AA}-0-0, 7_{LL23/24AA,LL66/67AA,Y92A}-0-0 and 7_{LL36/37AA}-0-0 localized to late endosomes/lysosomes. In contrast, 7_{LL23/24AA,LL36/37AA,LL66/67AA}-0-0 did not colocalize with LAMP-1 but reached the plasma membrane, just like the 7_{LL23/24AA,LL36/37AA,}-0-0 chimera in Fig. 5B.

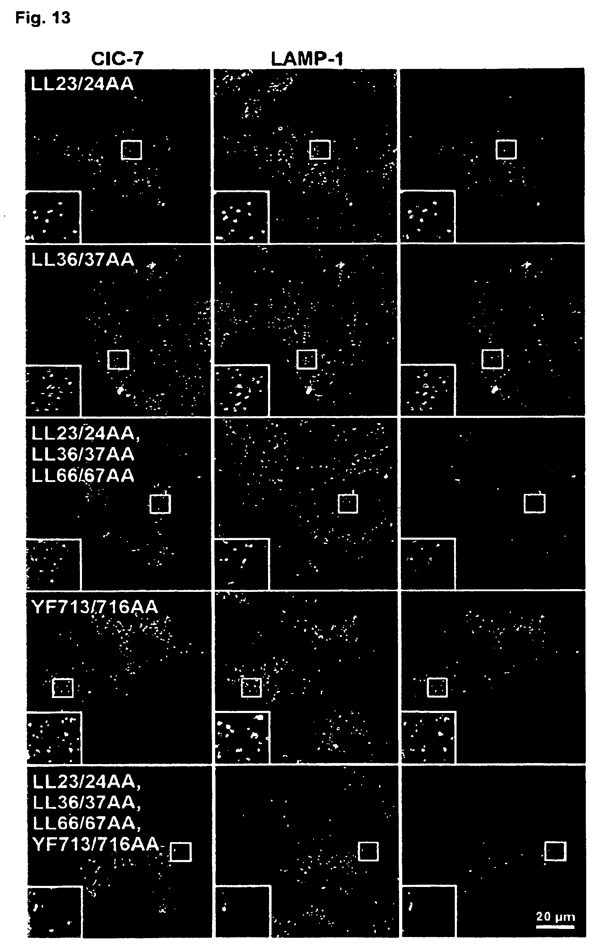

Fig. 13. Subcellular localization of further rCIC-7 mutants in transiently transfected HeLa cells, determined by immunostaining for CIC-7 (green in merge) and the late endosomal/lysosomal marker LAMP-1 (red in merge). While rCIC-7_{LL23/24AA}, rCIC-7_{LL36/37AA}, rCIC-7_{LL66/67AA} and rCIC-7_{YF713/716AA} almost completely colocalized with LAMP-1, rCIC-7LL23/24AA,LL36/37AA,LL66/67AA- and rCIC-7_{LL23/24AA,LL36/37AA,LL66/67AA,YF713/716AA}-transfected cells displayed CIC-7 staining at the plasma membrane in addition to colocalization with LAMP-1.

Fig. 14. Depolarisation-induced currents in oocytes expressing mutated CIC-7. Current/voltage curve of a *Xenopus* oocyte injected with cRNAs encoding rCIC-7^{LL23/24AA,LL36/37AA} and mOstm1 as measured by two-electrode voltage-clamp.

Fig. 15. Model of cell-based assay. The overexpressed, plasma membrane-localised sodium channel FaNaC is activated by application of its ligand, the peptide FMRFamide (a). Due to the sodium gradient across the plasma membrane (lower [Na⁺] inside the cell) opening of FaNaC leads to influx of sodium ions (b), rendering the inside-negative membrane potential, Ψ, positive (c). This depolarisation results in an activation of overexpressed CIC-7*/Ostm1 (*indicating its being mutated), which in turn leads to an influx of chloride ions coupled to an efflux of protons (d). Intracellular [Cl⁻] or even better pH can be measured with fluorescent dyes.

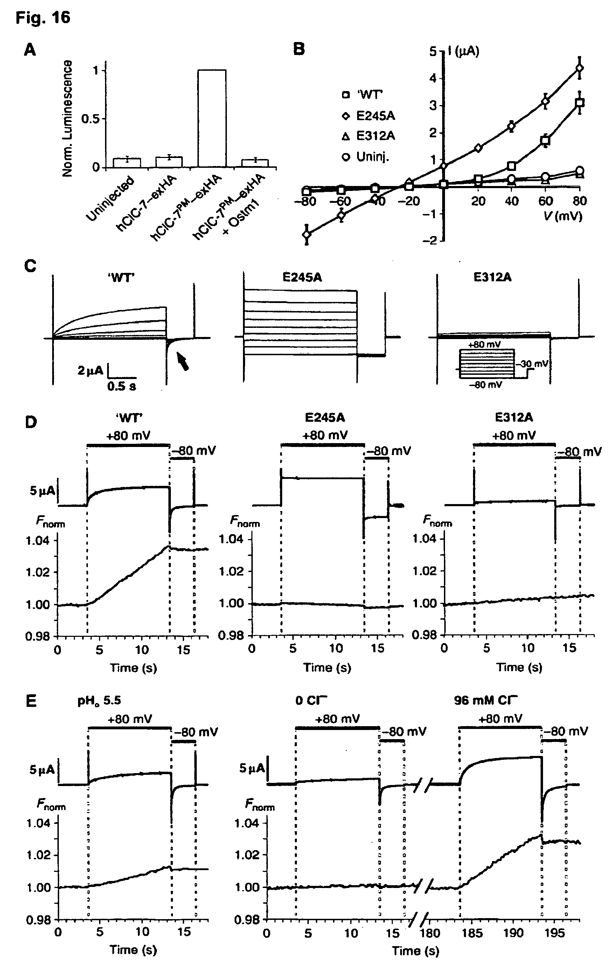

Fig. 16 Basic characterization of CIC-7PM /Ostm1 in Xenopus oocytes. (A) Chemiluminescence assay for surface detection of hCIC-7 and hCIC-7PM with an extracytosolic HA tag. hCIC-7PM-exHA, but not hCIC-7-exHA is detected at the surface of Xenopus oocytes. Co-expression with Ostm1 suppresses the luminescence signal although CIC-7PM /Ostm1 yields plasma membrane currents. Mean luminescence intensity (error bars, s.e.m.) normalized to hCIC-7PM -exHA from four independent experiments. (B, C) Two-electrode voltage-clamp analysis in Xenopus oocytes. Representative voltage-clamp traces (C) of rCIC-7PM ('WT'), rCIC-7PM (E245A) and rCIC-7PM (E312A) co-expressed with Ostm1. Arrow indicates tail currents. Voltage was clamped from _80 to Þ 80 mV in 2 s steps of 20 mV (inset). Mean ± s.e.m. of currents reached after 2 s plotted (B) as function of voltage (rCIC-7PM , n = 20; rCIC-7PM (E245A), n = 13; rCIC-7PM (E312A), n = 11; uninjected, n = 16 oocytes from at least three batches). Virtually identical results were obtained with hCIC-7PM. (D, E) Intracellular pH changes of Xenopus oocytes co-expressing rCIC-7PM ('WT' or glutamate mutants) with Ostm1 in response to a 10-s depolarization. Top traces, clamp currents; bottom traces, pH-dependent BCECF fluorescence measured with the Fluorocyte method (Zdebik et al, 2008). Increased fluorescence means alkalinization. Unless indicated otherwise, extracellular solution contained 96 mM CI- at pH 7.4. For 0 CI-, gluconate replaced Cl-. Left traces in (D) and (E) are from the same oocyte and centre and right recordings in (E) are from one oocyte as well. Similar results were obtained with at least five oocytes from three batches.

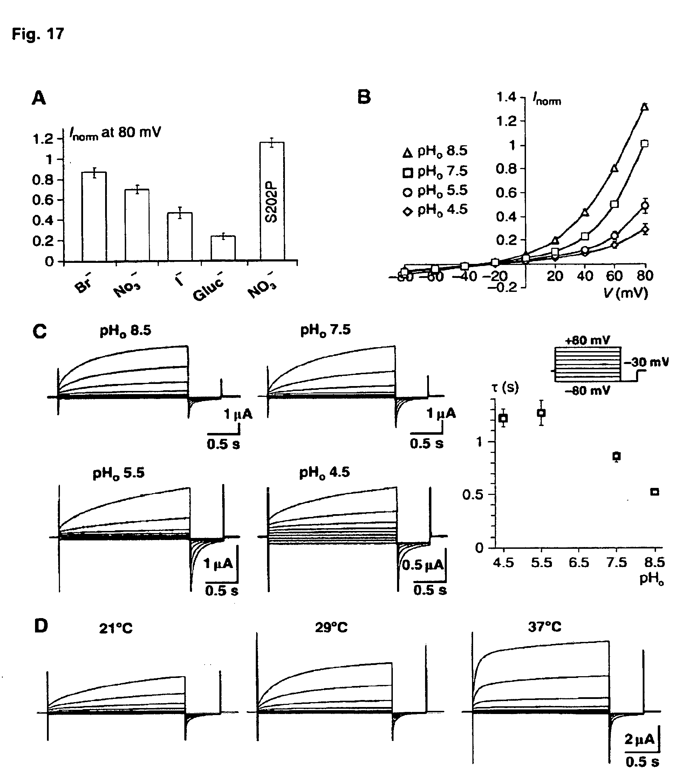

Fig. 17 Modulation of CIC-7/Ostm1 by anions, protons and temperature. (A) Relative anion conductance of oocyte-expressed rCIC-7PM /Ostm1 in the presence of different extracellular anions (96 mM). Clamp protocol as in Figure 1C. Mean ± s.e.m. of currents reached after 2 s at Þ 80 mV was normalized to the current in Cl- for each oocyte (white bars) (Cl, n = 32 oocytes; Br, n = 7; NO3, n = 6; I , n = 5; gluconate (glue), n = 5). Grey bar, NO3 conductance of rCIC-7PM (S202P)/Ostm1 mutant measured and normalized as above (n = 9). (B) Dependence of rCIC-7PM /Ostm1 currents on pHo. I/V curves were obtained as in Figure 1B with currents normalized to those at pHo= 7.4 and 80 mV; X6 oocytes per data point. (C) Left, typical voltage-clamp traces (top right, protocol) obtained at different pHovalues. Note different current scales that were chosen to normalize current amplitudes to the end of Þ 80 mV pulse for better visualizing changes in activation kinetics. Right, t was determined by single-exponential fit of the 80-mV traces for X6 oocytes per pH value. Mean ± s.e.m. as function of pHo. (D) Typical voltageclamp traces of rCIC-7PM /Ostm1 (protocol as in (C)) at different temperatures, representative for 11 oocytes in which temperature was changed between 21, 29 deg C (n = 8) and/or 37 deg C (n = 9).

### EXAMPLES

The following examples serve to demonstrate certain of embodiments of the invention but do not represent a limiting definition.

### RESULTS

*Binding* of *endosomal sorting machinery to N- and C-terminal cytosolic domains of endosomal*/*lysosomal CLCs -* To screen for interactions of intracellular CLCs with the endosomal/lysosomal transport machinery, we generated recombinant GST fusion proteins of their cytosolic amino- and carboxy-terminal domains (NT and CT, respectively) and of the cytosolic CT of the CIC-7 Beta-subunit Ostm1, a protein with a single transmembrane span. In addition, we included the cytosolic domains of the plasma membrane-residing channel CIC-1 and GST alone. Pull-down experiments from HeLa cell lysate were performed with equal amounts of the purified, immobilized fusion protein (Fig. 8). Immunoblotting of bound protein against subunits of all four AP complexes, GGA proteins and clathrin revealed numerous interactions between the CLC domains and sorting machinery components (Fig. 1). Pull-down experiments from homogenates of mouse kidney and brain yielded similar binding patterns for clathrin and AP complexes (not shown). Interactions with GGAs, however, could not be probed in those tissues as our antibodies did not recognize the mouse proteins.

CIC-1 interacted through its N-terminus with AP2 (Fig. 1A). CIC-3-NT bound clathrin, as reported previously (23). In contrast to this previous study, we did not detect binding of AP-1 or -2 to CIC-3 (Fig. 1A). Neither did CIC-3 fusion proteins bind AP-3, as would have been expected from the reported role of AP-3 in targeting CIC-3 (3). CIC-4 bound clathrin weakly with its Nterminus (Fig. 1A), but no binding to AP or GGA proteins was found with either its N- or Cterminus (Fig. 1A,B). CIC-5-NT pulled down AP- 1 and AP-2 as well as clathrin (Fig. 1A), whereas none of the investigated proteins were bound by the CIC-5 C-terminus (Fig. 1 B). The N-terminus of CIC-6 interacted weakly with AP-2 and more strongly with AP-3 (Fig. 1A). AP-3 also bound to the CIC-6 C-terminus, albeit less strongly (Fig. 1B). Both N- and C-termini of CIC-7 bound AP-2 and AP-3 (Fig. 1A,B,C) and its N-terminus also bound AP-1 (Fig. 1A,C). AP-1 and AP-4 binding to the C-terminus of CIC-7 appeared weak because it was not detected in all pull-downs (compare Fig.1B and Fig. 2G). The N-terminus of CIC-7 also pulled down GGA2 more efficiently than GGA3 (Fig. 1A) and GGA1 (not shown). The C-terminus of the CIC-7 Beta-subunit Ostm1 bound AP-2 and AP-3, but neither GGA2 nor GGA3 (Fig. 1C). These interactions were also found when pulldown experiments were performed with lysates of murine adult fibroblasts lacking CIC-7 (39) or Ostm1 (38) (not shown), excluding a possible indirect interaction of Ostm1-CT mediated by bound CIC-7 and *vice versa.*

*Identification of the sorting motifs mediating clathrin and adaptor binding -* To investigate the interactions in more detail, we set out to identify the respective binding motifs. The N- and C-termini of CLCs contain numerous potential tyrosine-based or dileucine sorting motifs that might mediate the binding of APs or GGAs (Fig. 2A). To test for the actual role of the potential motifs in intracellular CLCs, we replaced the tyrosines of tyrosine-based motifs and the leucines (or leucine-isoleucines) of dileucine motifs, respectively, by alanines in our GST fusion proteins and tested their impact on adaptor binding in pull-down experiments.

The N-terminal domain of CIC-3, which bound clathrin but not APs or GGAs, contains a potential tyrosine-based motif at Tyr₂₇ (Y₂₇DDF, Fig. 2A). However, previous work (23) had shown that clathrin did not bind to this motif, but to a more amino-terminal stretch containing acidic residues and two dileucines (L₁₃LDLLDE, Fig. 2A). Our pull-down experiments confirmed that the strong binding of CIC-3-NT to clathrin (Fig. 1A) was virtually abolished when all four leucines of this stretch were replaced by alanines (Fig. 2B). Within the N-terminus of CIC-5, which bound AP-1, AP-2 and clathrin, there is one potential tyrosine-based motif at Tyr₁₄ (Y₁₄DDF). It is conserved in the N-termini of CIC-3 and CIC- 4 (Fig. 2A), which, however, lack detectable AP binding (Fig. 1A). We therefore tested initially another sequence in CIC-5-NT (ESTWALI₄₈) that almost matches the (DE)XXXL(LI) consensus sequence (Fig. 2A). However, mutating Leu₄₇Ile₄₈ to alanines did not significantly reduce the binding of APs and clathrin to CIC-5-NT, whereas surprisingly mutating Tyr₁₄ to Ala strongly reduced these interactions (Fig. 2C). Combining Y14A with the L47A/I48A double mutation did not reduce binding further (Fig. 2C).

The N-terminus of CIC-6, which bound AP-2 and AP-3 (Fig. 1A), contains three potential tyrosine-based motifs (Y₄₈ESL, Y₆₁LEV, and Y₇₆EAV) (Fig. 2A). Because the crystal structure of the prokaryotic EcCIC-1 protein suggests that the third motif might extend into the first membrane-spanning helix B (43), we focused on the first two motifs. Mutating Tyr₄₈ to Ala strongly reduced AP-2 and AP-3 binding, with a weaker effect seen with the Y61A mutant (Fig. 2D). Combining both mutations virtually abolished binding (Fig. 2D). Recently, the basic amino acid stretch K₇₁KGRR (Fig. 2A) was implicated in the localization of heterologously expressed CIC-6 to early and/or recycling endosomes, an effect that may involve lipid rafts (37). When this stretch had been mutated to A₇₁AGAA, CIC-6 was trafficked to late endosomes/lysosomes (37). Introducing this mutation in our GST-CIC-6-NT seemed to affect binding of AP-2 and AP-3 only slightly (Fig. 2D). Despite the low efficiency with which the C-terminus of CIC-6 pulled down AP-3 (Fig. 1B), it displays many potential sorting motifs (Fig. 2A). The EKEDLL₇₀₆ sequence conforms to the [DE]XXXL[LI] consensus motif for AP binding, while DLTLL₇₉₅ conforms to the DXXLL consensus sequence for GGA-binding. Both sequences are located in the stretch between the two cystathione Beta-synthetase (CBS) domains (46,47) which is particularly long in CIC-6. In this interdomain stretch there are also three tyrosine-based motifs (Y₇₁₇PNL, Y₇₇₄AEM, and Y₇₈₄PDI). Such a motif is also found at the very end of the protein (Y₈₆₆QTI). Another potential Y-based motif (Y₈₅₅EFL) is predicted to be positioned within an α-helix of the CBS2 domain and is not conserved in mouse (where the Y is substituted by N). We therefore did not investigate it further. We next replaced the key tyrosines and leucines, respectively, of the other motifs by alanines. Mutants in which the extreme C-terminal Y₈₆₆QTI motif was disrupted (Y866A), either by itself or together with mutations of other potential sorting motifs, did not bind AP-3 anymore (Fig. 2E, Fig. 9A). In contrast, various mutants in which the Y₈₆₆QTI motif was preserved (Fig. 2E), even that with all of Leu₇₀₅Leu₇₀₆, Tyr₇₁₇, Tyr₇₇₄, Tyr₇₈₄ and Leu₇₉₄Leu₇₉₅ substituted with alanines (Fig. 9A), still pulled down AP-3 similar to the WT C-terminus. We conclude that specific binding of AP-3 to the CIC-6 C-terminus depends on the Y₈₆₆QTI motif at the extreme C-terminus. In comparison to the N-terminal fusion protein, however, AP-3 binding to CIC-6-CT is weak. The N-terminus of human CIC-7 contains a DXXLL consensus sequence for GGA binding (DDELL₆₉) (Fig. 2A) which might underlie the binding of GGA2 and GGA3 (Fig. 1A). The binding of AP-1, AP-2 and AP-3 might be mediated by the potential [DE]XXXL[LI]-type motif EAAPLL₂₄ and/or the tyrosine-based motif with Y₉₄ESL (Fig. 2A). Indeed, mutating leucines Leu₆₈ and Leu₆₉ to alanines specifically abolished the interaction with the GGA proteins, leaving AP binding unaffected (Fig. 2F). Conversely, replacing Leu₂₃ and Leu₂₄ by alanines abolished the pull-down of APs, but not of GGAs. We therefore did not examine a putative role of the Y₉₄ESL 'motif' in AP binding. As expected, combined disruption of both dileucine motifs inhibited binding of both APs and GGAs (Fig. 2F). The only conventional AP binding motif in the Cterminus of CIC-7 is a canonical YXXΦ) motif at Tyr₇₁₅ (Y₇₁₅PRF, Fig. 2A). However, mutating this tyrosine to alanine did not reduce the binding of AP complexes to CIC-7-CT (Fig. 2G). Although it weakly bound AP-2 and AP-3, the cytosolic C-terminus of Ostm1 does not display any consensus tyrosine-based or dileucine motifs. To narrow down the position of the binding site we generated two overlapping constructs with the first part of the C-terminus (Ser₃₀₇-Thr₃₂₈) and the extreme C-terminal part (Ile₃₁₉-Thr₃₃₈), respectively, both fused to GST. The sequence between Ser₃₀₇ and Thr₃₂₈ was sufficient to pull down AP-2 and AP-3 as efficiently as the full C-terminus, whereas no binding to the C-terminal fragment Ile₃₁₉-Thr₃₃₈ was detected (Fig. 2H). The interaction was not dependent on the dipeptide LI₃₁₉ (Fig. 2H).

*Infernalization of heterologously expressed CIC-5 is largely independent of its N-terminal AP-2-binding motif -* The Y₁₄DDF motif in the N-terminus of CIC-5 binds AP-2 (and other adaptors, Figs. 1A, 2C) and might therefore be involved in its endocytosis from the plasma membrane. To test this hypothesis, we introduced the Y14A mutation into a full-length CIC-5 construct and expressed it in CHO cells which have negligible endogenous levels of CIC-5 (48). Immunofluorescence microscopy did not reveal obvious differences in the subcellular localization between WT and Y14A CIC-5 (Fig. 3A). Both proteins displayed some plasma membrane localization in addition to intracellular vesicular staining. We tested whether we could detect stronger plasma membrane staining with a mutant in the C-terminal 'PY-like' internalization motif (34) (Fig. 2A). This motif mediates E3 ubiquitin ligase-mediated internalization of CIC-5 in heterologous expression systems (34,35). However, we could not observe an increase in plasma membrane expression of this Y672E mutant by immunofluorescence (Fig. 3A). Even twofold increases in plasma membrane expression are difficult to detect by immunofluorescence. For a more reliable, quantitative comparison of cell surface expression between WT and mutants, we introduced an HA epitope into the extracytosolic loop between helices B and C of CIC-5 (34). As negative control we appended an HA-tag to the cytosolic Cterminus. All constructs were expressed to similar extents in *Xenopus* oocytes (Fig. 3B). The extracellular presence of the epitope was quantified using an HA-antibody in a chemiluminescence assay (34,45). These experiments revealed that the surface expression of CIC-5_{Y14A} and WT CIC-5 were indistinguishable, whereas the PY mutant CIC- 5_{Y672E} showed a roughly twofold higher surface expression (Fig. 3C) as in our previous work (34). Likewise two-electrode voltage-clamp measurements of oocytes expressing the Y672E mutant yielded about 2-fold higher currents than those expressing either WT or Y14A CIC-5 (Fig. 3D). We conclude that unlike the C-terminal PQPPY₆₇₂VRL motif, the N-terminal Y₁₄DDF motif does not play a significant role in plasma membrane localization of CIC-5.

*CIC-6 sorting through its cytosolic domains -* To investigate the role of the sorting motifs identified in CIC-6, we compared the subcellular localization of full-length WT with mutant CIC-6 in transfected HeLa cells. As reported previously (37), heterologously expressed CIC-6 colocalized with the recycling-endosome marker transferrin receptor (TfR) (Fig. 4A) rather than with markers of late endosomes (not shown). This contrasts with the late endosomal localization of native CIC-6 (36). When the tyrosines of the confirmed AP3-binding Y₈₆₆QTI motif and of the Y₄₈ESL, Y₆₁LEV consensus sequences were changed to alanine, either alone or in combination, the localization of the resultant mutant was not altered (not shown). We therefore mutated all potential 'classical' sorting motifs in the N- and Ctermini of CIC-6 (Fig. 2A) in combination. However, even this protein (CIC- 6_{Y48A,Y61A,LL705/706AA,Y717A,Y774A,Y784A,LL794/795AA,Y866A}) was targeted to TfR-positive endosomes of transfected cells like WT CIC-6 (Fig. 4A). We then introduced into this heavily mutated construct two other point mutations (W590A,E591A) to disrupt a potential unconventional sorting signal reported to mediate lysosomal targeting (49). These two residues are located at the end of the last intramembrane helix R and had not been included in the fusion proteins used for pull-down experiments (Fig. 2A). However, even these additional mutations did not change the colocalization of CIC-6 with the TfR (not shown). We finally generated chimeric proteins in which portions of CIC-6 were replaced by equivalent segments of the plasma membrane Cl⁻ channel CIC-0 from *Torpedo marmorata* (42). Cytoplasmic N-terminal and C-terminal domains and the central transmembrane domain were assembled in different combinations and their subcellular localization was determined in transfected HeLa cells (Fig. 4B). When both N- and C-termini of CIC-6 were replaced in chimera 0-6-0 by those of CIC-0, predominant ER-like staining was observed in less than 50% of transfected cells. In the majority of cells the 0-6-0 chimera was strongly plasma membrane-localized just like CIC-0 itself (Fig. 4B), demonstrating the importance of the cytosolic domains for endosomal sorting.

Unfortunately, chimeras possessing the CIC-6 C-terminus and the Nterminus of CIC-0 (0-6-6 and 0-0-6) did not leave the ER (Fig. 4B). The two chimeras with an Nterminus of CIC-6 and the C-terminus of CIC-0 (6- 6-0 and 6-0-0) localized to intracellular punctate structures (Fig. 4B) where they colocalized with the TfR (not shown) just as heterologously expressed CIC-6. Hence the N-terminus of CIC-6 is sufficient for endosomal targeting. Unexpectedly, the chimera 6-0-6 displayed a perinuclear localization pattern (Fig. 4B) and colocalized with the Golgi protein GM130 (Fig. 10A). Disrupting the C-terminal Y₈₆₆QTI AP-3 binding site of CIC-6 by the Y866A mutation did not affect the apparent ER localization of 0-6-6 and 0-0-6 (not shown), nor the perinuclear localization of 6-0-6 (Fig. 10B) even when combined with mutations Y48A and Y61A which together virtually abolished binding of AP-2 and AP-3 to the CIC-6 N-terminus (Fig. 2D).

*Sorting motifs responsible for the subcellularlocalization of CIC-7*/*Ostm1 -* To investigate the role in lysosomal sorting of CIC-7 of identified AP- and GGA-binding motifs, we transfected HeLa cells with rat CIC-7 (rCIC-7) or with chimeras between rCIC-7 and CIC-0. We did not co-transfect the Beta-subunit Ostm1 because Ostm1 bound APs in our pull-down experiments and therefore might have confounded our results. Transfected full-length rCIC-7 nearly perfectly colocalized with the late endosomal/lysosomal marker protein LAMP-1 (Fig. 5A) like observed previously in native cells (40). When we replaced the cytosolic N- and C-terminal regions of rCIC-7 by those of CIC-0, the resulting chimera 0-7-0 yielded a predominantly reticular staining pattern indicative of ER retention (Fig. 5A). However, a small proportion of 0-7-0 reached the plasma membrane. Unlike WT CIC-7, the chimera did not colocalize with LAMP-1 (Fig. 5A).

The N-terminus of rCIC-7 suffices to direct the plasma membrane Cl- channel to late endosomes and lysosomes, as revealed by the colocalization of 7-0-0 with LAMP-1 in transfected cells (Fig. 5B). When we disrupted in this construct the N-terminal EGAPLL₂₄ and DDELL₆₇ motifs (homologous to EAAPLL₂₄ and DDELL₆₉ in human (Fig. 11), which bind APs and GGAs, respectively (Fig. 2F)), either alone (Fig. 12) or in combination (Fig. 5B), the mutant 7- 0-0 chimeras were still sorted to late endosomes/lysosomes. Additionally mutating tyrosine Tyr₉₂ (homologous to human Tyr₉₄ in the YESL₉₄ motif that was not involved in AP binding (Fig. 2F)) did not alter this localization (Fig. 12).

Compared to human CIC-7, rCIC-7 exhibits an additional [DE]XXXL[LI] consensus motif (EETPLL₃₇) that is a candidate site for AP protein binding (Fig. 11). Replacing both leucines of this motif by alanines did not affect the subcellular localization of 7-0-0, whereas the combined disruption of all three amino-terminal dileucine motifs brought the 7-0-0 chimera to the plasma membrane (Fig. 12). Surprisingly, disruption of the GGA-binding motif DDELL₆₇ was not required for this effect, as also the mutant in which only both consensus sites for AP-binding were disrupted (7_{LL23/24AA,LL36/37AA}-0-0) reached the cell surface instead of colocalizing with LAMP-1 (Fig. 5B).

Further experimentation also reveals that mutation of the single dileucine to dialanine (LL23/24AA) of the human CIC-7 protein leads to cell-surface localization of the CIC-7. In addition to this finding, it can be shown that modification of the human CIC-7 protein, to a protein carrying the mutations LL23/24AA and LL68/69AA (hCIC-7PM) partially traffics to the plasma membrane, as ascertained in a chemiluminescence assay for an added extracytosolic HA tag (Figure 16). This assay failed to detect hCIC-7PM when co-expressed with Ostm1, possibly owing to a shielding of the epitope by the highly glycosylated N-terminus of Ostm1. Indeed, both rCIC-7PM /Ostm1 and hCIC-7PM /Ostm1 gave robust plasma membrane currents.

We next explored the role of these N-terminal dileucine motifs for the targeting of fulllength rCIC-7. Introducing those mutations that directed the 7-0-0 chimera to the plasma membrane (Fig. 5B, Fig. 12) into rCIC-7 resulted in mutants that resided to some extent in the plasma membrane (rCIC-7LL23/24AA,LL36/37AA (Fig. 6) and rCIC-7LL23/24AA,LL36/37AA_{,LL66/67AA} (Fig. 13)). In further agreement with results for the 7-0-0 chimera, neither the individual disruption of the three N-terminal dileucine motifs (not shown, Fig. 13), nor the combined disruption of the EGAPLL₂₄ and DDELL₆₇ motifs (Fig. 6) changed the localization of CIC-7. The remaining partial colocalization of these mutants with LAMP-1 cannot be attributed to signals remaining in the mutated CIC-7 N-terminus because these mutations completely abolished the late endosomal/lysosomal localization of the 7-0-0 chimera (Fig. 5B). Since the 0-7-0 chimera (Fig. 5A) suggests that this localization is neither owed to the transmembrane part, it is probably the C-terminus, which bound AP adaptors in our pull-down experiments (Fig. 1 B), that provides additional cues for endosomal/lysosomal sorting.

Although disruption of the only tyrosine-based consensus motif (Y₇₁₅PRF₇₁₈) in the C-terminal GST fusion protein of human CIC-7 did not interfere with binding of APs (Fig. 2G), we mutated the homologous Tyr₇₁₃ and Phe₇₁₆ in full-length rCIC-7. This mutant (rCIC-7_{YF713/716AA}) remained localized to late endosomes/lysosomes (Fig. 13). When this mutation was added on top of those combinations that already partially shifted the constructs to the cell surface, the resulting rCIC-7_{LL23/24AA,LL36/37AA,}Y_{F713/716AA} (not shown) and rCIC- 7_{LL23/24AA,LL36/37AA,LL66/67AA,YF713/716AA} (Fig. 13) still displayed the partial colocalization with LAMP-1 in addition to their presence at the plasma membrane. Obviously the YPRF motif of CIC-7 is not responsible for the apparent ability of the CIC-7 C-terminus to partially direct CIC-7 to lysosomes when N-terminal lysosomal trafficking signals have been disrupted.

CIC-7 is required for ER export of its Beta- subunit, Ostm1, but CIC-7 is targeted to lysosomes even in the absence of Ostm1 (38). As we found weak binding of APs to the CIC-7 Beta-subunit Ostm1 (Fig. 1C, 2H), we wondered whether Ostm1 could support lysosomal sorting of CIC-7 mutants whose dominant lysosomal targeting sequences had been disrupted. To this end, we transiently co-transfected Ostm1 bearing a C-terminal green fluorescent protein (GFP)-tag (Ostm1-GFP) with either WT or sorting mutants of rCIC-7. Co-expression of both WT rCIC-7 and rCIC-7_{LL23/24AA,LL36/37AA} were sufficient to ensure ER export of Ostm1. In both cases Ostm1-GFP. colocalized with the CIC-7 construct (Fig. 7). With WT CIC-7, Ostm1-GFP was sorted to late endosomes/lysosomes, whereas it strongly labeled the cell surface in addition to a partial lysosomal localization when co-transfected with rCIC- 7_{LL23/24AA,LL36/37AA}. Thus, CIC-7 determines the localization of Ostm1.

Since currents of human and rat CIC-7PM were indistinguishable in both Xenopus oocytes and transfected mammalian cells, CIC-7PM may refer to both variants. The following experiments provide further characterization of the plasma membrane-localized CIC-7 transporters and therefore further evidence and support for the method of screening candidate molecules as disclosed herein.

Expression of CIC-7PM /Ostm1 in Xenopus oocytes (Figure 16B and C), tsA201 or HeLa cells yielded strongly outwardly rectifying currents that activated slowly at voltages more positive than B Þ 20 mV. In stark contrast to CIC-3 through CIC-6, full activation was not even observed after several seconds and slow deactivation resulted in tail currents at negative voltages (Figure 16C (arrow)). Whole-cell patch-clamp experiments in HeLa cells showed that CIC-7PM /Ostm1 currents do not require intracellular ATP. We neither observed significant changes in current amplitudes like described for CIC-5 (Zifarelli and Pusch, 2009b) which is known to bind ATP by its CBS domains (Meyer et al, 2007), nor changes in voltage dependence. As typical for CLC antiporters (Friedrich et al, 1999; Li et al, 2002; Dutzler et al, 2003; Picollo and Pusch, 2005; Scheel et al, 2005; Zdebik et al, 2008; Bergsdorf et al, 2009; Neagoe et al, 2010), mutating the 'gating glutamate' (E245 in rat) of CIC-7PM to alanine resulted in almost ohmic, time independent currents, and changing the 'proton glutamate' (E312 in rat) to alanine reduced currents to background levels (Figure 16B and C). CIC-7PM / Ostm1 mediated Cl-/H+ exchange as evident from depolarization-induced intracellular alkalinization of Xenopus oocytes expressing these proteins (Figure 16D). In these 'Fluorocyte' experiments, the pH-dependent fluorescence of BCECF previously injected into oocytes provides a semiquantitative measure of cytosolic pH changes in response to depolarizing voltage steps. Depolarization not only activates CIC-7PM /Ostm1 but also provides a driving force for coupled H+ exit/Cl- entry. Outward transport of protons required extracellular CI- (Figure 16E), could occur against its electrochemical gradient (pH = 5.5, Figure 16E), and was abolished by either the E245A or the E312A mutation (Figure 16D). CIC-7PM /Ostm1 currents decreased upon replacing extracellular CI- by I-, but unlike CIC-4 and CIC-5 (Friedrich et al, 1999), currents were not larger with NO3- (Figure 17A). Replacing a CI- -coordinating serine by proline (rCIC-7 (S202P)) increased the nitrate/chloride conductance ratio as with other CLC antiporters (Bergsdorf et al, 2009; Zifarelli and Pusch, 2009a; Neagoe et al, 2010) and with CIC-0 (Bergsdorf et al, 2009; Picollo et al, 2009). Akin to CIC-4, CIC-5 and CIC-6 (Friedrich et al, 1999; Neagoe et al, 2010; Picollo et al, 2010), currents were decreased by acidic extracellular pH (Figure 17B). In addition to a diminished driving force for CI- /H+ -exchange with increased extracellular [H+], faster activation kinetics at more alkaline pHo contributes to the pH dependence of CIC-7PM /Ostm1 outward currents (Figure 17C). Voltage-dependent current activation was also strongly dependent on temperature (Figure 17D). Mono-exponential fits yielded activation rate constants of 2.8±0.2 s⁻1 at 21 deg C and 16.6±1.9 s⁻1 at 37 deg C, giving an estimate of Q10=3.

Despite the pivotal role of CLC Cl-/H+- exchangers in endosomal/lysosomal function, it has remained enigmatic how their differential localization to the various endosomal/lysosomal compartments is achieved. We used GST fusion proteins of the amino- and carboxy-terminal cytosolic domains of all intracellular CLCs to systematically test and compare their interactions with clathrin and its adaptors, AP-1 through -4 and GGA proteins. The resulting interaction pattern did not depend on the source of cell lysates used for the pull-down (HeLa cells, mouse brain or kidney) and agreed well with the subcellular localization of the various CLC proteins (Fig. 1D). For example, AP-3, which mediates cargo sorting for transport to late endosomes, interacted specifically with late endosomal CIC-6 and lysosomal CIC-7/Ostm1 whereas it was not bound by the other CLCs which localize to earlier endosomal compartments or the plasma membrane. On the other hand, the AP-2 adaptor complex involved in endocytosis from the plasma membrane was strongly bound by CIC-5 which cycles between endosomes and the cell surface. The amino-acid sequences of cytoplasmic CLC domains suggested the presence of consensus binding motifs. In several cases, N- or C-terminal domains displayed more than one candidate binding site. For instance, the N-terminus of CIC-7 contains sites for both AP and GGA binding, and the N-terminus of CIC-6 displays several sites for AP binding which may be functionally redundant to some degree. Candidate binding sites were validated experimentally by disrupting them through mutagenesis, either individually or in combination. When introduced into the respective fusion protein, these mutations often abolished or reduced binding of adaptor proteins or clathrin, thereby confirming these motifs as being functionally relevant. In some cases, however, such mutations failed to affect binding. This situation is not unusual because the candidate binding site might be sterically inaccessible or may require more amino-acids than those specified in the consensus sequence. Moreover, even if a 'real' binding site had been disrupted by mutagenesis, the functional consequence might be masked by an additional, unidentified binding site in the fusion protein. Indeed, unconventional binding sites do exist and we were unable to identify the site(s) by which the C-termini of either CIC-7 or its Beta-subunit Ostm1 bound AP-2 and AP- 3.

Whereas the binding of specific adaptor proteins to the various CLC transporters agreed well with their intracellular localization (Fig. 1 D), it often proved difficult to demonstrate their involvement in the intracellular trafficking of CLC proteins. Even when all confirmed adaptor binding sites in the N- and C-termini of CIC-6 were disrupted by mutagenesis, the heavily mutated CIC-6 was still trafficked to TfR-positive recycling endosomes just like transfected WT CIC-6. One has to realize, however, that this localization is abnormal. Native CIC-6 is found in late endosomes of neurons, the only cells significantly expressing this CLC protein (36). Since the CIC-6 mRNA is rather ubiquitously expressed (50), CIC-6 might require a neuronspecific Beta-subunit for its stability, similar to CIC-K Cl- channels which are unstable without their Beta-subunit barttin (51) or like CIC-7 which needs Ostm1 (38). In contrast to Ostm1, which is not needed for the lysosomal localization of CIC-7 (38), barttin plays a crucial role in targeting CIC-K channels to the plasma membrane (18). Likewise, a so far unknown Beta-subunit for CIC-6 might traffic CIC-6 to late endosomes. If so, our study addressed a situation that is not found *in vivo.* Nonetheless, the present 6-0-0 chimera showed that the N-terminus of CIC-6 contains endosomal targeting signals. Unfortunately, we obtained ambiguous localization results (not shown) when we disrupted the confirmed N-terminal AP binding sites in this chimeric construct. We are therefore unable to state with confidence that those motifs play a role in CIC-6 sorting.

The situation is much clearer with the late endosomal/lysosomal CIC-7/Ostm1 heteromer (38). Although AP-1 and AP-2 bound weakly to an unidentified binding site in its carboxyterminus, Ostm1 did not significantly influence the localization of CIC-7 in transfected cells, agreeing with our previous work (38). Even when the disruption of lysosomal sorting signals in CIC-7 led to a partial mislocalization of the transporter to the plasma membrane, co-expression with Ostm1 did not increase the proportion of CIC-7/Ostm1 found in late endosomes/lysosomes. Likewise, and also agreeing with our previous work (38), co-transfection with WT CIC-7 trafficked Ostm1 to late endosomes/lysosomes. Importantly, CIC-7 mutants that mislocalized to the plasma membrane carried Ostm1 to that domain as well. Hence, the subcellular localization of Ostm1 seems to depend entirely on sorting signals in CIC-7, and Ostm1 lacks an effect on CIC-7 trafficking.

Both N- and C-termini of CIC-7 strongly bound AP-3, an adaptor involved in trafficking to late endosomes. Subsequent transport to lysosomes does not require further sorting. The CIC-7 N-terminus also bound GGA proteins which might direct CIC-7 to early endosomes from where it would be sorted to late endosomes by AP-3. The prominent role in lysosomal sorting of the CIC-7 N-terminus was revealed by a chimera in which it replaced the N-terminus of the plasma membrane Cl- channel CIC-0. The resulting chimera 7-0-0 was targeted to late endosomes/lysosomes rather than to the plasma membrane. Strong lysosomal targeting signals are provided by the two dileucine AP-binding motifs present in the rat CIC-7 Nterminus. When these two motifs were disrupted together, the mutated 7-0-0 chimera was found in the plasma membrane like CIC-0. Somewhat surprisingly, the GGA binding site did not seem important for lysosomal sorting. Full-length CIC-7 could be partially directed to the plasma membrane by disrupting just those two AP binding motifs. However, a large proportion of the mutant remained in late endosomes/lysosomes to which it was probably directed by its AP-3 binding carboxy-terminus. As disruption of the only 'conventional' candidate AP binding site in the Cterminus had no effect, we were unable to fully direct CIC-7 to the plasma membrane with a few point mutations. Nonetheless, the partially plasmamembrane localized CIC-7 mutant that carries just four point mutations in the cytoplasmic Nterminus should prove useful for characterizing its biophysical properties.

With the notable exception of the Nterminus of CIC-5, which bound AP-2 (and clathrin) to a site which we confirmed by mutagenesis, the N- and C-termini of CIC-3 through CIC-5 did not bind APs or GGAs in our pull-down experiments. Although AP-2 binding to CIC-5 would fit well with the assumed recycling of CIC-5 over the plasma membrane, the disruption of its binding motif did not increase its abundance in the plasma membrane. Hence other mechanisms must be operating in directing these endosomal CLCs to their respective compartments. One such mechanism may be binding to clathrin as described previously for CIC-3 (23). In addition, there might be binding sites in the cytoplasmic aspect of the membranespanning parts of CLC proteins, an issue we could not investigate with our pull-down experiments. Indeed, a tyrosine-based motif between intramembrane helices D and E has recently been implicated in the rapid recycling of the Cl- channel CIC-2 between the plasma membrane and an endosomal compartment (17). On the other hand, CIC-3, -4, and -5 may heterodimerize (4,52) akin to the previously described heteromer formation between plasma membrane CLC channels (44,53), and sorting signals present in one of the subunits may determine the trafficking of the heterodimer. In summary, we newly identified several AP and GGA binding sites in the cytoplasmic parts of vesicular CLC anion/proton exchangers. The known roles of confirmed binding partners in facilitating specific sorting steps agreed well with the native subcellular localization of the CLCs they bound to. In several cases, however, these interactions are not the only ones that direct vesicular CLCs to their normal destination, because no change in localization was observed when the respective binding sites were disrupted. Those cases where vesicular CLC proteins could be directed to the plasma membrane with a few point mutations, however, should provide excellent opportunities to study their biophysical properties in detail.

### METHODS

The following methods and materials were used in the examples of the present invention. These methods do not represent a limiting description of the invention. One skilled in the art is aware of alternatives and other modes of carrying out the present invention.

*Antibodies -* For immunoblotting we used mouse monoclonal antibodies directed against the adaptor protein subunits γ (AP-1), Beta2 and μ2 (both AP-2), Delta, μ3A and σ3 (all AP-3), and ε (AP-4), and against GGA2, GGA3 (all from BD Bioscience), and α-tubulin (clone DM1A, Sigma), and the rabbit antibody 5A2 against CIC-5 (29). Clathrin heavy chain (CHC) was detected with culture supernatant of the hybridoma cell line X22 (ATCC CRL-2228). HRP-tagged secondary antibodies were purchased from Dianova. For immunostaining of cells, we used monoclonal mouse antibodies directed against transferrin receptor (Invitrogen) and LAMP-1 (clone H4A3, DSHB) and rabbit antibodies against CIC-5 (5A2 (29)), CIC-6 (6N2 (36), 6C3 (41)), CIC-7 (7N4B (40)) and against a carboxy-terminal fusion protein of CIC-0 (Steinmeyer and Jentsch, unpublished). The latter had not been affinity-purified, which may explain the higher background in nonexpressing cells in Figs. 4 and 5. No signal above background staining was observed when constructs not possessing the CIC-0 carboxyterminus had been transfected (not shown). Secondary antibodies conjugated to AlexaFluor 488, 546 or 633 were from MolecularProbes.

*Expression constructs -* To generate GST fusion constructs for the C-terminus of CIC-7, the sequence between amino acids (aa) 616-805 of human CIC-7 was amplified by PCR and cloned into the expression vector pETM30 (EMBL, protein expression and purification core facility). For the other GST fusion constructs of the CLC amino- and carboxy-termini (NT and CT), respectively, the sequences encoding the following amino acids of the respective human CLC were inserted into pGEX-5X-1 (GE Healthcare) resulting in an N-terminal GST-tag: CIC-1-NT, aa 1-117; CIC-1-CT, aa 592-989; CIC-3-NT, aa 1-67; CIC-3-CT, aa 586-760; CIC-4-NT, aa 1-67; CIC-4- CT, aa 584-760; CIC-5-NT, aa 1-54; CIC-5-CT, aa 573-746; CIC-6-NT, aa 1-80; CIC-6-CT, aa 591- 869; CIC-7-NT, aa 1-126. For the GST fusion protein of the Ostm1 carboxy-terminus, the sequence encoding aa 307-338 of mouse Ostm1 has been cloned into pGEX-5X-1.

For cell culture expression, *Torpedo* CIC-0 (42) was subcloned into pcDNA3 (Invitrogen). Constructs for human CIC-5, human CIC-6 and rat CIC-7 in this vector have been described previously (40,41). For the generation of chimeric constructs containing parts of CIC-0 and either CIC-6 or CIC-7, the DNA sequences encoding the amino-terminal part (aa 1- 48 for CIC-0, aa 1-77 for CIC-6 and aa 1-123 for CIC-7), the transmembrane region (43) from the beginning of helix B until the end of helix R (aa 49-524 for CIC-0, aa 78-588 for CIC-6 and aa 124- 614 for CIC-7) and the carboxy-terminal region (aa 525-805 for CIC-0, aa 589-869 for CIC-6 and aa 615-805 for CIC-7) of the respective CLC were combined by recombinant PCR with overlapping primers and cloned into pcDNA3. For expression of fluorescently tagged Ostm1, the sequence encoding mouse Ostm1 was cloned into pEGFPN3 (Clontech) linking Ostm1 with the C-terminal green fluorescent protein (GFP) by the sequence VDGTAGPGSIAT.

To express hCIC-5 in *Xenopus* oocytes, the cDNA was cloned into pTLN (44). An HA epitope was inserted between amino acids Glu₁₀₇ and Val₁₀₈ (extra-cytosolic loop between helices B and C) or at the C-terminus by PCR mutagenesis. Point mutations were introduced by PCR with primers carrying the respective mutation. All constructs were confirmed by sequencing the complete ORF.

*GST pull-down -* GST fusion proteins were expressed in *Escherichia coli* (BL21, DE3) for 5-6 h at 25 °C after induction with 0.12 mg/ml isopropyl-beta-D-thiogalactopyranoside before pelleting the cells by centrifugation at 5000 g. Cells were lysed by sonification in PBS supplemented with 0.5 mg/ml AEBSF (Roche), protease inhibitor cocktail (Complete_{®}, Roche) and lysozyme (Sigma) and subsequent incubation with 1% (w/v) Triton X-100 on ice. GST fusion proteins were affinity-purified from a 20,000g supernatant by a 2h incubation with glutathion sepharose (GE Healthcare) under constant agitation at 4 °C and subsequent washing with PBS. Purity and concentration was estimated by Coomassie staining after SDS-PAGE with BSA as standard.

For a single pull-down experiment, 1.0 - 1.5 ml lysate of a 10-cm dish of confluent HeLa cells in PBS supplemented with 1 % (w/v) Triton X-100, 0.5 mg/ml AEBSF (Pefabloc SC, Roche), protease inhibitor cocktail (Complete_{®}, Roche) and 1 mM Na₃O₄ centrifuged at 10,000 g for 10 min and the supernatant was incubated with roughly 50 μg GST fusion protein coupled to sepharose for 2 h under constant agitation at 4 °C. After four washes with PBS supplemented with 0.1 % (w/v) Triton X-100, 0.5 mg/ml AEBSF (Roche) and protease inhibitor cocktail (Complete_{®}, Roche), bound protein was eluted by incubation in SDS sample buffer at 55 °C for 15 min. After sedimenting the sepharose beads, the eluate was separated by SDS-PAGE and probed by immunoblot. Pull-downs from mouse brain or kidney lysate were performed equivalently, with PBS replaced by HEPES-buffered saline (HBS). For tissue lysate preparation, two brains or four kidneys of WT C57BI/6 mice were homogenized in 10 ml HBS with 0.5 mg/ml AEBSF (Roche), protease inhibitor cocktail (Complete_{®}, Roche) and 1 mM Na₃VO₄. The supernatant of a 10min centrifugation at 10,000 g was supplemented with 1%_{final} Triton X-100. 1.5 ml of the supernatant of two further centrifugation steps (15 min at 10,000 g and 20 min at 50,000 g) was used per pull-down as described above.

*Expression in cell culture and fluorescence microscopy -* Plasmid DNA encoding the respective construct was transfected using FuGENE6 (Roche) according to the manufacturer's instruction and cells were grown in a humidified 5%-CO₂ incubator at 37 °C for further 24 to 48 h before fixation with 4% PFA in PBS for 15 min. For immunostaining, cells were incubated with 30 mM glycine in PBS for 5 min and permeabilized with 0.1 % saponin in PBS for 10 min. Both primary and AlexaFluor-coupled secondary (Molecular Probes) antibodies were applied in PBS/0.05% saponin supplemented with 3 % BSA. Images were acquired with an LSM510 laser scanning confocal μscope equipped with a 63x 1.4 NA oil-immersion lens (Zeiss).

*Voltage clamp analysis and surface expression assay in Xenopus laevis oocytes -* Capped cRNA was transcribed from constructs in pTLN linearized with *Mlu*I (extracellular HA) or Hpal (C-terminal HA) using the mMessage mMachine kit (Ambion) and SP6 polymerase. 20 ng cRNA were injected into defolliculated oocytes. Oocytes were kept at 17°C in ND96 (in mM: 96 NaCl, 2 KCI, 1.8 CaCl₂, 1 MgCl₂, 5 HEPES, pH 7.5) for two days before analysis. Two-electrode voltage-clamp measurements were performed at room temperature using a TurboTec10C amplifier (npi electronic) and pClamp10 software (Molecular Devices) as decribed previously (34). Surface expression of HA-tagged CIC-5 protein was determined as described previously (34,45).

*Patch-clamp experiments -* Whole-cell patch-clamp measurements on cultured cells used patch pipettes of 3-5 MO resistance filled with (in mM) 110 CsCl, 10 NaCl, 0.5 CaCl2, 1 EGTA, 2 MgATP, 40 HEPES, pH 7.2. The calculated free Ca2 Þ concentration was 180 nM. The bath solution contained (in mM) 130 NaCl, 5 KCI, 1 MgCl2, 1 CaCl2, 10 glucose, 20 HEPES, pH 7.4 with NaOH. Osmolarity was adjusted with sucrose to 280- 290 mOsmol/l for the pipette solution and to 300 mOsmol/l for the extracellular solution. To alter Cl and pH gradients, NaCl was replaced partially by Na-gluconate and pH was buffered with 20 mM Tris (pH 8.4) or 20 mM MES (pH 6.4). Data were acquired with an EPC-10 double amplifier and Pulse software (HEKA).

### REFERENCES

1. Jentsch, T. J. (2008) Crit. Rev. Biochem. Mol. Biol. 43, 3-36
2. Stobrawa, S. M., Breiderhoff, T., Takamori, S., Engel, D., Schweizer, M., Zdebik, A. A., Bösl, M. R., Ruether, K., Jahn, H., Draguhn, A., Jahn, R., and Jentsch, T. J. (2001) Neuron 29, 185-196
3. Salazar, G., Love, R., Styers, M. L., Werner, E., Peden, A., Rodriguez, S., Gearing, M., Wainer, B. H., and Faundez, V. (2004) J Biol Chem 279, 25430-25439
4. Suzuki, T., Rai, T., Hayama, A., Sohara, E., Suda, S., Itoh, T., Sasaki, S., and Uchida, S. (2006) J Cell Physiol 206, 792-798
5. Wartosch, L., Fuhrmann, J. C., Schweizer, M., Stauber, T., and Jentsch, T. J. (2009) FASEB J 23, 4056-4068
6. Jentsch, T. J. (2007) J Physiol 578, 633-640
7. Weinert, S., Jabs, S., Supanchart, C., Schweizer, M., Gimber, N., Richter, M., Rademann, J., Stauber, T., Kornak, U., and Jentsch, T. J. (2010) Science 328, 1401-1403
8. Novarino, G., Weinert, S., Rickheit, G., and Jentsch, T. J. (2010) Science 328, 1398-1401
9. Günther, W., Piwon, N., and Jentsch, T. J. (2003) Pflügers Arch 445, 456-462
10. Hara-Chikuma, M., Yang, B., Sonawane, N. D., Sasaki, S., Uchida, S., and Verkman, A. S. (2005) J Biol Chem 280, 1241-1247
11. Bonifacino, J. S., and Traub, L. M. (2003) Annu Rev Biochem 72, 395-447
12. Braulke, T., and Bonifacino, J. S. (2009) Biochim Biophys Acta 1793, 605-614
13. Robinson, M. S. (2004) Trends Cell Biol 14, 167-174
14. Bonifacino, J. S. (2004) Nat Rev Mol Cell Biol 5, 23-32
15. Hirst, J., Sahlender, D. A., Choma, M., Sinka; R., Harbour, M. E., Parkinson, M., and Robinson, M. S. (2009) Traffic 10, 1696-1710
16. Peña-Münzenmayer, G., Catalán, M., Cornejo, I., Figueroa, C. D., Melvin, J. E., Niemeyer, M. I., Cid, L. P., and Sepúlveda, F. V. (2005) J Cell Sci 118, 4243-4252
17. Cornejo, I., Niemeyer, M. I., Zuniga, L., Yusef, Y. R., Sepúlveda, F. V., and Cid, L. P. (2009) J Cell Physiol 221, 650-657
18. Estévez, R., Boettger, T., Stein, V., Birkenhäger, R., Otto, M., Hildebrandt, F., and Jentsch, T. J. (2001) Nature 414, 558-561
19. Staub, O., Gautschi, I., Ishikawa, T., Breitschopf, K., Ciechanover, A., Schild, L., and Rotin, D. (1997) EMBO J 16, 6325-6336
20. Miller, F. J., Jr., Filali, M., Huss, G. J., Stanic, B., Chamseddine, A., Barna, T. J., and Lamb, F. S. (2007) Circ Res 101, 663-671
21. Li, X., Wang, T., Zhao, Z., and Weinman, S. A. (2002) Am J Physiol Cell Physiol 282, C1483-C1491
22. Weylandt, K. H., Nebrig, M., Jansen-Rosseck, N., Amey, J. S., Carmena, D., Wiedenmann, B., Higgins, C. F., and Sardini, A. (2007) Mol Cancer Ther 6, 979-986
23. Zhao, Z., Li, X., Hao, J., Winston, J. H., and Weinman, S. A. (2007) J Biol Chem 282, 29022-29031
24. Maritzen, T., Keating, D. J., Neagoe, I., Zdebik, A. A., and Jentsch, T. J. (2008) J Neurosci 28, 10587-10598
25. Ogura, T., Furukawa, T., Toyozaki, T., Yamada, K., Zheng, Y. J., Katayama, Y., Nakaya, H., and Inagaki, N. (2002) FASEB J 16, S63-S65
26. Gentzsch, M., Cui, L., Mengos, A., Chang, X. B., Chen, J. H., and Riordan, J. R. (2003) J Biol Chem 278, 6440-6449
27. Mohammad-Panah, R., Harrison, R., Dhani, S., Ackerley, C., Huan, L. J., Wang, Y., and Bear, C. E. (2003) J Biol Chem 278, 29267-29277
28. Okkenhaug, H., Weylandt, K. H., Carmena, D., Wells, D. J., Higgins, C. F., and Sardini, A. (2006) FASEB J 20, 2390-2392
29. Günther, W., Lüchow, A., Cluzeaud, F., Vandewalle, A., and Jentsch, T. J. (1998) Proc Nat Acad Sci U S A 95, 8075-8080
30. Sakamoto, H., Sado, Y., Naito, I., Kwon, T. H., Inoue, S., Endo, K., Kawasaki, M., Uchida, S., Nielsen, S., Sasaki, S., and Marumo, F. (1999) Am J Physiol 277, F957-F965
31. Vandewalle, A., Cluzeaud, F., Peng, K. C., Bens, M., Lüchow, A., Günther, W., and Jentsch, T. J. (2001) Am J Physiol Cell Physiol 280, C373-C381
32. Wang, Y., Cai, H., Cebotaru, L., Hryciw, D. H., Weinman, E. J., Donowitz, M., Guggino, S. E., and Guggino, W. B. (2005) Am J Physiol Renal Physiol 289, F850-F862
33. Rickheit, G., Wartosch, L., Schaffer, S., Stobrawa, S., Novarino, G., Weinert, S., and Jentsch, T. J. (2010) J Biol Chem 285, 17595-17603
34. Schwake, M., Friedrich, T., and Jentsch, T. J. (2001) J Biol Chem 276, 12049-12054
35. Hryciw, D. H., Ekberg, J., Lee, A., Lensink, I. L., Kumar, S., Guggino, W. B., Cook, D. I., Pollock, C. A., and Poronnik, P. (2004) J Biol Chem 279, 54996-55007
36. Poët, M., Kornak, U., Schweizer, M., Zdebik, A. A., Scheel, O., Hoelter, S., Wurst, W., Schmitt, A., Fuhrmann, J. C., Planells-Cases, R., Mole, S. E., Hübner, C. A., and Jentsch, T. J. (2006) Proc Natl Acad Sci U S A 103, 13854-13859
37. Ignoul, S., Simaels, J., Hermans, D., Annaert, W., and Eggermont, J. (2007) PLoS ONE 2, e474
38. Lange, P. F., Wartosch, L., Jentsch, T. J., and Fuhrmann, J. C. (2006) Nature 440, 220-223
39. Kasper, D., Planells-Cases, R., Fuhrmann, J. C., Scheel, O., Zeitz, O., Ruether, K., Schmitt, A., Poët, M., Steinfeld, R., Schweizer, M., Kornak, U., and Jentsch, T. J. (2005) EMBO J 24, 1079-1091
40. Kornak, U., Kasper, D., Bösl, M. R., Kaiser, E., Schweizer, M., Schulz, A., Friedrich, W., Delling, G., and Jentsch, T. J. (2001) Cell 104, 205-215
41. Neagoe, I., Stauber, T., Fidzinski, P., Bergsdorf, E. Y., and Jentsch, T. J. J Biol Chem 285, 21689-21697
42. Jentsch, T. J., Steinmeyer, K., and Schwarz, G. (1990) Nature 348, 510-514
43. Dutzler, R., Campbell, E. B., Cadene, M., Chait, B. T., and MacKinnon, R. (2002) Nature 415, 287-294
44. Lorenz, C., Pusch, M., and Jentsch, T. J. (1996) Proc Natl Acad Sci U S A 93, 13362-13366
45. Zerangue, N., Schwappach, B., Jan, Y. N., and Jan, L. Y. (1999) Neuron 22, 537-548
46. Bateman, A. (1997) Trends Biochem Sci 22, 12-13
47. Ponting, C. P. (1997) J Mol Med 75, 160-163
48. Steinmeyer, K., Schwappach, B., Bens, M., Vandewalle, A., and Jentsch, T. J. (1995) J Biol Chem 270, 31172-31177
49. Piccirillo, R., Palmisano, I., Innamorati, G., Bagnato, P., Altimare, D., and Schiaffino, M. V. (2006) J Cell Sci 119, 2003-2014
50. Brandt, S., and Jentsch, T. J. (1995) FEBS Lett 377, 15-20
51. Rickheit, G., Maier, H., Strenzke, N., Andreescu, C. E., De Zeeuw, C. I., Muenscher, A., Zdebik, A. A., and Jentsch, T. J. (2008) EMBO J 27, 2907-2917
52. Mohammad-Panah, R., Ackerley, C., Rommens, J., Choudhury, M., Wang, Y., and Bear, C. E. (2002) J Biol Chem 277, 566-574
53. Weinreich, F., and Jentsch, T. J. (2001) J Biol Chem 276, 2347-2353
54. Graves, A. R., Curran, P. K., Smith, C. L., and Mindell, J. A. (2008) Nature 453, 788-792

## Claims

1. CIC-7 protein that is presented on the cell surface and/or plasma membrane, **characterised in that** interaction between CIC-7 and the endosomal and/or lysosomal sorting machinery is inhibited and/or disrupted, whereby
the CIC-7 protein is selected from human, rat or mouse CIC-7 protein, wherein said CIC-7 protein comprises an N-terminal sequence according to SEQ ID NO: 19, 20 or 21, which is modified by a mutation in the following AP or GGA recognition motifs:
- In the human CIC-7 sequence the 19-EAAPLL-24 motif (SEQ ID NO: 2) and/or the 65-DDELL-69 motif (SEQ ID NO: 8),
- In the mouse and/or rat CIC-7 sequences the 19-EGAPLL-24 motif (SEQ ID NO: 4), the 32-EETPLL-37 motif (SEQ ID NO: 6) and/or the 63-DDELL-67 motif (SEQ ID NO: 10),
wherein said CIC-7 protein comprises at least a mutation in the 19-EAAPLL-24 motif (SEQ ID NO: 2) in human CIC-7 or 19-EGAPLL-24 motif (SEQ ID NO: 4) in rat or mouse CIC-7, wherein rat or mouse CLC-7 additionally comprises a mutation in the 32-EETPLL-37 motif (SEQ ID NO: 6).

2. CIC-7 protein according to claim 1, **characterised in that** the mutation of AP or GGA recognition motifs comprise the LL23/24AA mutation in the human, mouse or rat CIC-7 sequence, the LL36/37AA mutation in the mouse or rat CIC-7 sequence, the LL66/67AA mutation in the mouse or rat CIC-7 sequence, the LL68/69AA mutation in the human CIC-7 sequence, the YF715/718AA mutation in the human CIC-7 sequence and/or the YF713/716AA mutation in the mouse or rat CIC-7 sequence.

3. CIC-7 protein according to at least one of the preceding claims, **characterised in that** the amino acid sequence comprises:
a) a sequence selected from the group comprising SEQ ID NO: 1, 3, 5, 7, 13, 14, 15, 16, 17 and 18, preferably from the group comprising SEQ ID NO: 13, 14, 15, 16, 17 and 18.
b) a sequence having at least 80% sequence identity, preferably 90% sequence identity, to the sequence according to a).

4. A method for screening a test compound for activity in modulating chloride and/or proton transport via CIC-7 **characterised in that**
- the CIC-7 protein according to any one of claims 1 to 3 is present on the cell surface and/or plasma membrane of a cell, whereby the protein is physically accessible to said test compound,
- said test compound is brought into contact with said cell, and
- proton transport and/or chloride transport, preferably via CIC-7, is measured,
- and optionally, that one or more adaptor proteins (AP) or Golgi-localized, γ-ear containing, Arf-binding adaptor proteins (GGA) are functionally disrupted and/or sequestered.

5. Method according to claim 4, **characterised in that**
the method is a cell-based screening assay.

6. Method according to at least one of claims 4 or 5, **characterised in that** the activity of the test compound in modulating chloride and/or proton transport via CIC-7 is measured by the change in transport of chloride ions across the cell membrane and/or the change in intracellular pH of a treated cell, preferably using pH-sensitive and/or chloride-sensitive detection agents, such as colouring agents.

7. Method according to at least one of claims 4 to 6, **characterised in that**
- Means for depolarising the cell are present, preferably that a ligand-gated sodium channel, preferably FaNaC, is co-expressed with the CIC-7 protein in the cell, or a sodium ionophore is present, and/or
- Ostm1 protein is co-expressed with the CIC-7 protein in the cell.

8. Nucleic acid molecule that encodes the CiC-7 protein according to any one of claims 1 to 3.

9. Vector comprising the nucleic acid molecule according to claim 8.

10. Cell comprising the nucleic acid molecule and/or vector according to claims 8 or 9.

11. Kit for carrying out the method according to claims 4 to 7, comprising the protein according to claims 1 to 3, the nucleic acid molecule and/or vector according to claims 8 and/or 9 and/or the cell according to claim 10, in addition to means for measuring the change in transport of chloride ions across the cell membrane and/or the change in intracellular pH or chloride concentration of a treated cell, preferably using pH-sensitive and/or chloride-sensitive detection agents, such as colouring agents.

## Patentansprüche

1. CIC-7-Protein, das auf der Zelloberfläche und/oder Plasmamembran präsentiert wird, **dadurch gekennzeichnet, dass** Interaktion zwischen CIC-7 und der endosomalen und/oder lysosomalen Sortiermaschinerie gehemmt und/oder gestört wird, wobei
das CIC-7-Protein aus Human-, Ratten- oder Maus-CIC-7-Protein ausgewählt ist, wobei das CIC-7-Protein eine N-terminale Sequenz gemäß SEQ ID NO: 19, 20 oder 21 umfasst, die durch eine Mutation in den folgenden AP- oder GGA-Erkennungsmotiven modifiziert ist:
- in der Human-CIC-7-Sequenz des 19-EAAPLL-24-Motivs (SEQ ID NO: 2) und/oder des 65-DDELL-69-Motivs (SEQ ID NO: 8),
- in den Maus- und/oder Ratten-CIC-7-Sequenzen des 19-EGAPLL-24-Motivs (SEQ ID NO: 4), des 32-EETPLL-37-Motivs (SEQ ID NO: 6) und/oder des 63-DDELL-67-Motivs (SEQ ID NO: 10),
wobei das CIC-7-Protein mindestens eine Mutation im 19-EAAPLL-24-Motiv (SEQ ID NO: 2) in Human-CIC-7 oder im 19-EGAPLL-24-Motiv (SEQ ID NO: 4) in Ratten- oder Maus-CIC-7 umfasst, wobei Ratten- oder Maus-CIC-7 zusätzlich eine Mutation im 32-EETPLL-37-Motiv (SEQ ID NO: 6) umfasst.

2. CIC-7-Protein nach Anspruch 1, **dadurch gekennzeichnet, dass** die Mutation von AP- oder GGA-Erkennungsmotiven die LL23/24AA-Mutation in der Human-, Maus- oder Ratten-CIC-7-Sequenz, die LL36/37AA-Mutation in der Maus- oder Ratten-CIC-7-Sequenz, die LL66/67AA-Mutation in der Maus- oder Ratten-CIC-7-Sequenz, die LL68/69AA-Mutation in der Human-CIC-7-Sequenz, die YF715/718AA-Mutation in der Human-CIC-7-Sequenz und/oder die YF713/716AA-Mutation in der Maus- oder Ratten-CIC-7-Sequenz umfasst.

3. CIC-7-Protein nach mindestens einem der vorhergehenden Ansprüche, **dadurch gekennzeichnet, dass** die Aminosäuresequenz Folgendes umfasst:
a) eine Sequenz, ausgewählt aus der Gruppe umfassend SEQ ID NO: 1, 3, 5, 7, 13, 14, 15, 16, 17 und 18, vorzugsweise aus der Gruppe umfassend SEQ ID NO: 13, 14, 15, 16, 17 und 18.
b) eine Sequenz mit mindestens 80 % Sequenzidentität, vorzugsweise 90 % Sequenzidentität, zu der Sequenz gemäß a).

4. Verfahren zum Screenen einer Testverbindung auf Aktivität beim Modulieren von Chlorid-und/oder Protonentransport über CIC-7, **dadurch gekennzeichnet, dass**
- das CIC-7-Protein nach einem der Ansprüche 1 bis 3 auf der Zelloberfläche und/oder Plasmamembran einer Zelle vorhanden ist, wobei das Protein der Testverbindung physikalisch zugänglich ist,
- die Testverbindung mit der Zelle in Kontakt gebracht wird, und
- Protonentransport und/oder Chloridtransport, vorzugsweise über CIC-7, gemessen wird,
- und wahlweise, dass ein oder mehrere AP-Proteine (Adapterproteine) oder GGA-Proteine (Golgi-localized, γ-ear containing, Arf-binding adapter proteins) funktionell gestört und/oder sequestriert werden.

5. Verfahren nach Anspruch 4, **dadurch gekennzeichnet, dass** das Verfahren ein zellbasierter Screening-Assay ist.

6. Verfahren nach mindestens einem der Ansprüche 4 oder 5, **dadurch gekennzeichnet, dass** die Aktivität der Testverbindung beim Modulieren des Chlorid-und/oder Protonentransports über CIC-7 durch die Änderung des Transports von Chloridionen durch die Zellmembran und/oder die Änderung des intrazellulären pH-Werts einer behandelten Zelle gemessen wird, vorzugsweise unter Verwendung von pH-empfindlichen und/oder chloridempfindlichen Detektionsmitteln, wie etwa Färbemitteln.

7. Verfahren nach mindestens einem der Ansprüche 4 bis 6, **dadurch gekennzeichnet, dass**
- Mittel zum Depolarisieren der Zelle vorhanden sind, vorzugsweise, dass ein ligandengesteuerter Natriumkanal, vorzugsweise FaNaC mit dem CIC-7-Protein in der Zelle coexprimiert wird, oder ein Natrium-Ionophor vorhanden ist, und/oder
- Ostm1 Protein mit dem CIC-7-Protein in der Zelle coexprimiert wird.

8. Nukleinsäuremolekül, das für das CIC-7-Protein nach einem der Ansprüche 1 bis 3 codiert.

9. Vektor, umfassend das Nukleinsäuremolekül nach Anspruch 8.

10. Zelle, umfassend das Nukleinsäuremolekül und/oder den Vektor nach den Ansprüchen 8 oder 9.

11. Kit zur Durchführung des Verfahrens nach den Ansprüchen 4 bis 7, umfassend das Protein nach Ansprüchen 1 bis 3, das Nukleinsäuremolekül und/oder den Vektor nach den Ansprüchen 8 und/oder 9 und/oder die Zelle nach Anspruch 10, zusätzlich zu Mitteln zum Messen der Änderung des Transports von Chloridionen durch die Zellmembran und/oder der Änderung des intrazellulären pH-Werts oder der Chloridkonzentration einer behandelten Zelle, vorzugsweise unter Verwendung von pH-empfindlichen und/oder chloridempfindlichen Detektionsmitteln, wie etwa Färbemitteln.

## Revendications

1. Protéine CIC-7 qui est présentée sur la surface cellulaire et/ou la membrane plasmatique, **caractérisée en ce que** l'interaction entre la CIC-7 et la machinerie de tri endosomale et/ou lysosomale est inhibée et/ou interrompue, moyennant quoi
la protéine CIC-7 est choisie parmi une protéine CIC-7 humaine, de rat ou de souris, ladite protéine CIC-7 comprenant une séquence N-terminale selon la SEQ ID N° 19, 20 ou 21, qui est modifiée par une mutation dans les motifs suivants AP ou GGA de reconnaissance :
- dans la séquence CIC-7 humaine, le motif 19-EAAPLL-24 (SEQ ID N°2) et/ou le motif 65-DDELL-69 (SEQ ID N° 8),
- dans les séquences CIC-7 de souris et/ou de rat, le motif 19-EGAPLL-24 (SEQ ID N°4), le motif 32-EETPLL-37 (SEQ ID N° 6) et/ou le motif 63-DDELL-67 (SEQ ID N° 10),
ladite protéine CIC-7 comprenant au moins une mutation dans le motif 19-EAAPLL-24 (SEQ ID N°2) dans la CIC-7 humaine ou dans le motif 19-EGAPLL-24 (SEQ ID N° 4) dans une CIC-7 de rat ou de souris, la CIC-7 de rat ou de souris comprenant de surcroît une mutation dans le motif 32-EETPLL-37 (SEQ ID N° 6).

2. Protéine CIC-7 selon la revendication 1, **caractérisée en ce que** la mutation des motifs de reconnaissance AP ou GGA comprend la mutation LL23/24AA dans la séquence de CIC-7 humaine, de souris ou de rat, la mutation LL36/37AA dans la séquence de CIC-7 de souris ou de rat, la mutation LL66/67AA dans la séquence de CIC-7 de souris ou de rat, la mutation LL68/69AA dans la séquence de CIC-7 humaine, la mutation YF715/718AA dans la séquence de CIC-7 et/ou la mutation YF713/716AA dans la séquence de CIC-7 de souris ou de rat.

3. Protéine CIC-7 selon au moins une des revendications précédentes, **caractérisée en ce que** la séquence d'acides aminés comprend :
a) une séquence choisie dans le groupe constitué des SEQ ID N° 1, 3, 5 ,7, 13, 14, 15, 16, 17 et 18, de préférence dans le groupe constitué des SEQ ID N° 13, 14, 15, 16, 17 et 18.
b) une séquence comptant au moins 80 % d'identité de séquence, de préférence 90 % d'identité de séquence, vis-à-vis de la séquence selon la revendication a).

4. Procédé de criblage d'un composé d'essai pour activité dans la modulation de transport de chlorure et/ou de protons par CIC-7, **caractérisé en ce que**
- la protéine CIC-7 conforme à l'une quelconque des revendications 1 à 3 est présente sur la surface cellulaire et/ou la membrane plasmatique d'une cellule, moyennant quoi la protéine est physiquement accessible audit composé d'essai,
- ledit composé d'essai est mis en contact avec ladite cellule, et
- le transport de protons et/ou le transport de chlorure, de préférence par CIC-7, est mesuré,
- et en option, qu'au moins une protéine d'adaptation (AP) ou des protéines adaptatrices localisées sur des cellules de Golgi, contenant une oreille γ, à liaison à Arf (GCA) sont fonctionnellement interrompues et/ou séquestrées.

5. Procédé selon la revendication 4, **caractérisé en ce que** le procédé est un essai de criblage basé sur des cellules.

6. Procédé selon l'une quelconque des revendications 4 ou 5, **caractérisé en ce que** l'activité du composé d'essai pour moduler le transport de chlorure et/ou de protons par CIC-7 se mesure par la variation du transport d'ions chlorure à travers la membrane cellulaire et/ou par la variation du pH intracellulaire d'une cellule traitée, de préférence grâce à des agents de détection sensibles au pH et/ou sensibles au chlorure, tels que des agents de coloration.

7. Procédé selon l'une quelconque des revendications 4 à 6, **caractérisé en ce que**
- des moyens de dépolarisation de la cellule sont présents, de préférence un canal sodium clôturé par un ligand, de préférence FaNaC, est coexprimé avec la protéine CIC-7 dans la cellule ou un ionophore sodium est présent, et/ou
- la protéine Ostm1 est coexprimée avec la protéine CIC-7 dans la cellule.

8. Molécule d'acide nucléique codant la protéine CIC-7 selon l'une quelconque des revendications 1 à 3.

9. Vecteur comprenant la molécule d'acide nucléique selon la revendication 8.

10. Cellule contenant la molécule d'acide nucléique et/ou le vecteur selon les revendications 8 ou 9.

11. Kit de réalisation du procédé selon les revendications 4 à 7, contenant la protéine selon les revendications 1 à 3, la molécule d'acide nucléique et/ou le vecteur selon les revendications 8 et/ou 9 et/ou la cellule selon la revendication 10, en plus du moyen de mesure de la variation du transport d'ions chlorure à travers la membrane cellulaire et/ou la variation du pH intracellulaire ou la concentration en chlorure d'une cellule traitée, utilisant de préférence des agents de détection sensibles au pH et/ou sensibles au chlorure, tels que des agents de coloration.


### SEQUENCE LISTING

<110> MAX-DELBRÜCK-CENTRUM FÜR MOLEKULARE MEDIZIN
   Forschungsverbund Berlin e.V.
   Jentsch, Thomas
   Stauber, Tobias
<120> CHLORIDE TRANSPORTER CLC-7 AND CELL-BASED SCREENING METHOD
<130> XII 648/11
<150> EP 10075351.6
   <151> 2010-08-16
<160> 21
<170> PatentIn version 3.5
<210> 1
   <211> 6
   <212> PRT
   <213> Homo sapiens
<400> 1
   Glu Ala Ala Pro Ala Ala
   1               5
<210> 2
   <211> 6
   <212> PRT
   <213> Homo sapiens
<400> 2
   Glu Ala Ala Pro Leu Leu
   1               5
<210> 3
   <211> 6
   <212> PRT
   <213> Rattus norvegicus
<400> 3
   Glu Gly Ala Pro Ala Ala
   1               5
<210> 4
   <211> 6
   <212> PRT
   <213> Rattus norvegicus
<400> 4
   Glu Gly Ala Pro Leu Leu
   1               5
<210> 5
   <211> 6
   <212> PRT
   <213> Rattus norvegicus
<400> 5
   Glu Glu Thr Pro Ala Ala
   1 5
<210> 6
   <211> 6
   <212> PRT
   <213> Rattus norvegicus
<400> 6
   Glu Glu Thr Pro Leu Leu
   1 5
<210> 7
   <211> 5
   <212> PRT
   <213> Homo sapiens
<400> 7
   Asp Asp Glu Ala Ala
   1 5
<210> 8
   <211> 5
   <212> PRT
   <213> Homo sapiens
<400> 8
   Asp Asp Glu Leu Leu
   1 5
<210> 9
   <211> 5
   <212> PRT
   <213> Rattus norvegicus
<400> 9
   Asp Asp Glu Ala Ala
   1 5
<210> 10
   <211> 5
   <212> PRT
   <213> Rattus norvegicus
<400> 10
   Asp Asp Glu Leu Leu
   1 5
<210> 11
   <211> 4
   <212> PRT
   <213> Rattus norvegicus
<400> 11
   Ala Pro Arg Ala
   1
<210> 12
   <211> 4
   <212> PRT
   <213> Rattus norvegicus
<400> 12
   Tyr Pro Arg Phe
   1
<210> 13
   <211> 126
   <212> PRT
   <213> Homo sapiens
<400> 13
   Met Ala Asn Val Ser Lys Lys Val Ser Trp Ser Gly Arg Asp Arg Asp
   1               5                   10                  15
   Asp Glu Glu Ala Ala Pro Ala Ala Arg Arg Thr Ala Arg Pro Gly Gly
               20                  25                  30
   Gly Thr Pro Leu Leu Asn Gly Ala Gly Pro Gly Ala Ala Arg Gln Ser
           35                  40                  45
   Pro Arg Ser Ala Leu Phe Arg Val Gly His Met Ser Ser Val Glu Leu
       50                  55                  60
   Asp Asp Glu Ala Ala Asp Pro Asp Met Asp Pro Pro His Pro Phe Pro
   65                  70                  75                  80
   Lys Glu Ile Pro His Asn Glu Lys Leu Leu Ser Leu Lys Tyr Glu Ser
                   85                  90                  95
   Leu Asp Tyr Asp Asn Ser Glu Asn Gln Leu Phe Leu Glu Glu Glu Arg
               100                 105                 110
   Arg Ile Asn His Thr Ala Phe Arg Thr Val Glu Ile Lys Arg
           115                 120                 125
<210> 14
   <211> 124
   <212> PRT
   <213> Rattus norvegicus
<400> 14
   Met Ala Asn Val Ser Lys Lys Val Ser Trp Ser Gly Arg Asp Arg Asp
   1               5                   10                  15
   Asp Glu Glu Gly Ala Pro Ala Ala Arg Arg Thr Gly Gln Pro Asp Glu
               20                  25                  30
   Glu Thr Pro Ala Ala Asn Gly Ala Gly Pro Gly Ala Arg Gln Ser His
           35                  40                  45
   Ser Ala Leu Phe Arg Ile Gly Gln Met Asn Asn Val Glu Leu Asp Asp
       50                  55                  60
   Glu Ala Ala Asp Pro Glu Val Asp Pro Pro His Thr Phe Pro Lys Glu
   65                  70                  75                  80
   Ile Pro His Asn Glu Lys Leu Leu Ser Leu Lys Tyr Glu Ser Leu Asp
                   85                  90                  95
   Tyr Asp Asn Ser Glu Asn Gln Leu Phe Leu Glu Glu Glu Arg Arg Ile
               100                 105                 110
   Asn His Thr Ala Phe Arg Thr Val Glu Ile Lys Arg
           115                 120
<210> 15
   <211> 124
   <212> PRT
   <213> Mus musculus
<400> 15
   Met Ala Asn Val Ser Lys Lys Val Ser Trp Ser Gly Arg Asp Arg Asp
   1               5                   10                  15
   Asp Glu Glu Gly Ala Pro Ala Ala Arg Arg Thr Gly Gln Pro Asp Glu
               20                  25                  30
   Glu Thr Pro Ala Ala Asn Gly Ala Gly Pro Gly Ala Arg Gln Ser His
           35                  40                  45
   Ser Ala Leu Phe Arg Ile Gly Gln Met Asn Asn Val Glu Leu Asp Asp
       50                  55                  60
   Glu Ala Ala Asp Pro Glu Val Asp Pro Pro His Thr Phe Pro Lys Glu
   65                  70                  75                  80
   Ile Pro His Asn Glu Lys Leu Leu Ser Leu Lys Tyr Glu Ser Leu Asp
                   85                  90                  95
   Tyr Asp Asn Ser Glu Asn Gln Leu Phe Leu Glu Glu Glu Arg Arg Ile
                   100                 105                 110
   Asn His Thr Ala Phe Arg Thr Val Glu Ile Lys Arg
           115                 120
<210> 16
   <211> 126
   <212> PRT
   <213> Homo sapiens
<400> 16
   Met Ala Asn Val Ser Lys Lys Val Ser Trp Ser Gly Arg Asp Arg Asp
   1               5                   10                  15
   Asp Glu Glu Ala Ala Pro Ala Ala Arg Arg Thr Ala Arg Pro Gly Gly
               20                  25                  30
   Gly Thr Pro Leu Leu Asn Gly Ala Gly Pro Gly Ala Ala Arg Gln Ser
           35                  40                  45
   Pro Arg Ser Ala Leu Phe Arg Val Gly His Met Ser Ser Val Glu Leu
       50                  55                  60
   Asp Asp Glu Leu Leu Asp Pro Asp Met Asp Pro Pro His Pro Phe Pro
   65                  70                  75                  80
   Lys Glu Ile Pro His Asn Glu Lys Leu Leu Ser Leu Lys Tyr Glu Ser
                   85                  90                  95
   Leu Asp Tyr Asp Asn Ser Glu Asn Gln Leu Phe Leu Glu Glu Glu Arg
               100                 105                 110
   Arg Ile Asn His Thr Ala Phe Arg Thr Val Glu Ile Lys Arg
           115                 120                 125
<210> 17
   <211> 124
   <212> PRT
   <213> Rattus norvegicus
<400> 17
   Met Ala Asn Val Ser Lys Lys Val Ser Trp Ser Gly Arg Asp Arg Asp
   1               5                   10                  15
   Asp Glu Glu Gly Ala Pro Ala Ala Arg Arg Thr Gly Gln Pro Asp Glu
               20                  25                  30
   Glu Thr Pro Ala Ala Asn Gly Ala Gly Pro Gly Ala Arg Gln Ser His
           35                  40                  45
   Ser Ala Leu Phe Arg Ile Gly Gln Met Asn Asn Val Glu Leu Asp Asp
   50 55 60
   Glu Leu Leu Asp Pro Glu Val Asp Pro Pro His Thr Phe Pro Lys Glu
   65 70 75 80
   Ile Pro His Asn Glu Lys Leu Leu Ser Leu Lys Tyr Glu Ser Leu Asp
   85 90 95
   Tyr Asp Asn Ser Glu Asn Gln Leu Phe Leu Glu Glu Glu Arg Arg Ile
   100 105 110
   Asn His Thr Ala Phe Arg Thr Val Glu Ile Lys Arg
   115 120
<210> 18
   <211> 124
   <212> PRT
   <213> Mus musculus
<400> 18
   Met Ala Asn Val Ser Lys Lys Val Ser Trp Ser Gly Arg Asp Arg Asp
   1 5 10 15
   Asp Glu Glu Gly Ala Pro Ala Ala Arg Arg Thr Gly Gln Pro Asp Glu
   20 25 30
   Glu Thr Pro Ala Ala Asn Gly Ala Gly Pro Gly Ala Arg Gln Ser His
   35 40 45
   Ser Ala Leu Phe Arg Ile Gly Gln Met Asn Asn Val Glu Leu Asp Asp
   50 55 60
   Glu Leu Leu Asp Pro Glu Val Asp Pro Pro His Thr Phe Pro Lys Glu
   65 70 75 80
   Ile Pro His Asn Glu Lys Leu Leu Ser Leu Lys Tyr Glu Ser Leu Asp
   85 90 95
   Tyr Asp Asn Ser Glu Asn Gln Leu Phe Leu Glu Glu Glu Arg Arg Ile
   100 105 110
   Asn His Thr Ala Phe Arg Thr Val Glu Ile Lys Arg
   115 120
<210> 19
   <211> 126
   <212> PRT
   <213> Homo sapiens
<400> 19
   Met Ala Asn Val Ser Lys Lys Val Ser Trp Ser Gly Arg Asp Arg Asp
   1               5                   10                  15
   Asp Glu Glu Ala Ala Pro Leu Leu Arg Arg Thr Ala Arg Pro Gly Gly
               20                  25                  30
   Gly Thr Pro Leu Leu Asn Gly Ala Gly Pro Gly Ala Ala Arg Gln Ser
           35                  40                  45
   Pro Arg Ser Ala Leu Phe Arg Val Gly His Met Ser Ser Val Glu Leu
       50                  55                  60
   Asp Asp Glu Leu Leu Asp Pro Asp Met Asp Pro Pro His Pro Phe Pro
   65                  70                  75                  80
   Lys Glu Ile Pro His Asn Glu Lys Leu Leu Ser Leu Lys Tyr Glu Ser
                   85                  90                  95
   Leu Asp Tyr Asp Asn Ser Glu Asn Gln Leu Phe Leu Glu Glu Glu Arg
               100                 105                 110
   Arg Ile Asn His Thr Ala Phe Arg Thr Val Glu Ile Lys Arg
           115                 120                 125
<210> 20
   <211> 124
   <212> PRT
   <213> Rattus norvegicus
<400> 20
   Met Ala Asn Val Ser Lys Lys Val Ser Trp Ser Gly Arg Asp Arg Asp
   1               5                   10                  15
   Asp Glu Glu Gly Ala Pro Leu Leu Arg Arg Thr Gly Gln Pro Asp Glu
               20                  25                  30
   Glu Thr Pro Leu Leu Asn Gly Ala Gly Pro Gly Ala Arg Gln Ser His
           35                  40                  45
   Ser Ala Leu Phe Arg Ile Gly Gln Met Asn Asn Val Glu Leu Asp Asp
       50                  55                  60
   Glu Leu Leu Asp Pro Glu Val Asp Pro Pro His Thr Phe Pro Lys Glu
   65                  70                  75                  80
   Ile Pro His Asn Glu Lys Leu Leu Ser Leu Lys Tyr Glu Ser Leu Asp
                   85                  90                  95
   Tyr Asp Asn Ser Glu Asn Gln Leu Phe Leu Glu Glu Glu Arg Arg Ile
                   100                 105                 110
   Asn His Thr Ala Phe Arg Thr Val Glu Ile Lys Arg
           115                 120
<210> 21
   <211> 124
   <212> PRT
   <213> Mus musculus
<400> 21
   Met Ala Asn Val Ser Lys Lys Val Ser Trp Ser Gly Arg Asp Arg Asp
   1               5                   10                  15
   Asp Glu Glu Gly Ala Pro Leu Leu Arg Arg Thr Gly Gln Pro Asp Glu
               20                  25                  30
   Glu Thr Pro Leu Leu Asn Gly Ala Gly Pro Gly Ala Arg Gln Ser His
           35                  40                  45
   Ser Ala Leu Phe Arg Ile Gly Gln Met Asn Asn Val Glu Leu Asp Asp
       50                  55                  60
   Glu Leu Leu Asp Pro Glu Val Asp Pro Pro His Thr Phe Pro Lys Glu
   65                  70                  75                  80
   Ile Pro His Asn Glu Lys Leu Leu Ser Leu Lys Tyr Glu Ser Leu Asp
                   85                  90                  95
   Tyr Asp Asn Ser Glu Asn Gln Leu Phe Leu Glu Glu Glu Arg Arg Ile
               100                 105                 110
   Asn His Thr Ala Phe Arg Thr Val Glu Ile Lys Arg
           115                 120